(19) **Europäisches Patentamt**
**European Patent Office**
**Office européen des brevets**

(11) Veröffentlichungsnummer: **0 089 637**
**B1**

(12) # EUROPÄISCHE PATENTSCHRIFT

(45) Veröffentlichungstag der Patentschrift: **15.10.86**

(51) Int. Cl.[4]: **C 07 D 209/42**, A 61 K 31/40

(21) Anmeldenummer: **83102679.4**

(22) Anmeldetag: **18.03.83**

(54) **Neue Derivate bicyclischer Aminosäuren, Verfahren zu ihrer Herstellung, diese enthaltende Mittel und deren Verwendung sowie neue bicyclische Aminosäuren als Zwischenstufen und Verfahren zu deren Herstellung.**

(30) Priorität: **23.03.82 DE 3210496**

(43) Veröffentlichungstag der Anmeldung:
**28.09.83 Patentblatt 83/39**

(45) Bekanntmachung des Hinweises auf die Patenterteilung:
**15.10.86 Patentblatt 86/42**

(84) Benannte Vertragsstaaten:
**AT BE CH DE FR GB IT LI LU NL SE**

(56) Entgegenhaltungen:
**EP-A-0 037 231**
**EP-A-0 049 658**
**EP-A-0 051 301**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Urbach, Hansjörg, Dr., Le Lavandoustrasse 41, D-6242 Kronberg/Taunus (DE)**
Erfinder: **Henning, Rainer, Dr., Völklinger Weg 56, D-6000 Frankfurt am Main 71 (DE)**
Erfinder: **Teetz, Volker, Dr., An der Tann 20, D-6238 Hofheim am Taunus (DE)**
Erfinder: **Wissmann, Hans, Dr., Falkenstrasse 12, D-6232 Bad Soden am Taunus (DE)**
Erfinder: **Becker, Reinhard, Dr., Adelheidstrasse 101, D-6200 Wiesbaden (DE)**

**EP 0 089 637 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1986

**Beschreibung**

Derivate bicyclischer Aminosäuren, die blutdrucksenkende Wirkung besitzen sind in EP-A-37 231 beschrieben. Die Erfindung betrifft neue Derivate der bicyclischen Aminosäuren der Formel I,

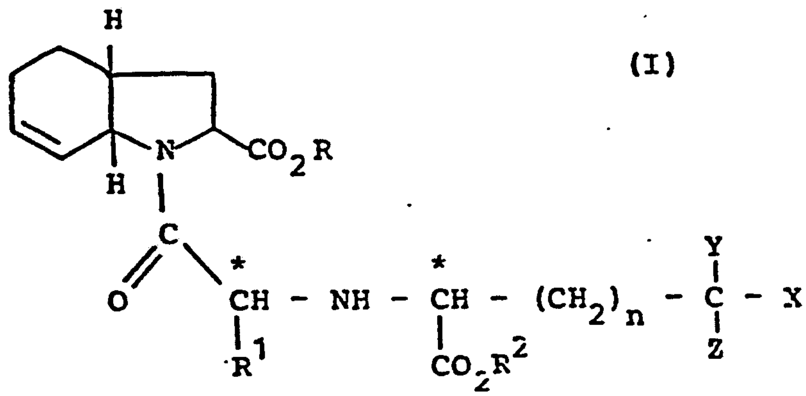

(I)

in der

n = 0 oder 1,

R = Wasserstoff, ($C_1$ bis $C_8$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_4$)-Acylamino, vorzugsw. ($C_1$ bis $C_4$)-Alkanoylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{10}$)-Aryl oder teilhydriertes ($C_6$ bis $C_{10}$)-Aryl, das jeweils durch ($C_1$ bis $C_4$)-Alkyl, ($C_1$oder $C_2$)-Alkoxy oder Halogen substituiert sein kann, ($C_6$ bis $C_{10}$)-Aryl-($C_1$ bis $C_4$)-alkyl oder ($C_7$ bis $C_{11}$)-Aroyl-$C_1$-alkyl die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, ($C_1$ bis $C_6$)-Alkyl, ($C_2$ bis $C_6$)-Alkenyl oder Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_9$)-Cycloalkyl, ($C_6$ bis $C_{10}$) Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino oder Methylendioxy mono-, di- oder trisubstituiert sein kann, oder 3-Indolyl bedeuten,

sowie deren physiologisch unbedenkliche Salze.

Als Salze kommen insbesondere Alkali- und Erdalkalisalze, Salze mit physiologisch verträglichen Aminen und Salze mit anorganischen oder organischen Säuren wie z.B. HCl, HBr, $H_2SO_4$, Maleinsäure, Fumarsäure in Frage.

Unter Aryl ist hier wie im folgenden vorzugsweise gegebenenfalls substituiertes Phenyl oder Naphthyl zu verstehen. Alkyl kann geradkettig oder verzweigt sein.

In der bevorzugten Konfiguration der H-Atome an C-3a und C-7a des Bicyclus kommen zwei mögliche Konfigurationen der Carboxygruppe in Betracht, und zwar die exo-Stellung (Formelrest Ia) und die endo-Stellung (Formelrest Ib) der Carboxygruppe.

Die endo-Stellung der Carboxygruppe an C-2 ist so definiert, daß die Carboxygruppe in Richtung des Sechsringes des Bicyclus, d.h. der konkaven Seite des Bicyclus zugewandt ist (Formelrest Ib).

Entsprechend ist die exo-Stellung der Carboxygruppe an C-2 so definiert, daß die Carboxygruppe in Richtung der betreffenden Brückenkopf-H-Atome orientiert ist (Formelrest Ia).

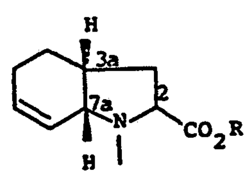

(I a)

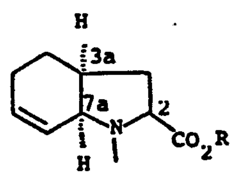

(I b)

Verbindungen der Formel I besitzen chirale C-Atome in den Positionen C-2, C-3a, C-7a, sowie in den mit einem Stern markierten C-Atomen der Seitenkette. Sowohl die R- als auch die S-Konfigurationen an allen Zentren sind Gegenstand der Erfindung. Die Verbindungen der Formel I können daher als optische Isomere, als

Diasteromere, als Racemate oder als Gemische derselben vorliegen. Bevorzugt sind jedoch die Verbindungen der Formel I, in denen das C-Atom 2 im bicyclischen Ringsystem, sowie die mit einem Stern (*) markierten C-Atome der Seitenkette S-Konfiguration aufweisen.

Besonders bevorzugte Verbindungen der Formel I sind solche, in denen

$n = 1$,

R = Wasserstoff oder Alkyl mit 1 bis 4 C-Atomen,

$R^1$ = Wasserstoff, ($C_1$ bis $C_3$)-Alkyl, ($C_2$ oder $C_3$)-Alkenyl Benzyl, Phenethyl, 4-Amino-butyl oder Benzoylmethyl,

$R^2$ = Wasserstoff, ($C_1$ bis $C_4$)-Alkyl oder Benzyl und

X = Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro oder Methylendioxy, mono- oder disubstituiert oder im Falle von Methoxy trisubstituiert sein kann, bedeuten,

insbesondere solche Verbindungen der Formel I, in denen $n=1$, R = Wasserstoff, $R^1$ = Methyl, X = Phenyl, $R^2$ = Wasserstoff oder Ethyl bedeuten, der Bicyclus die cis-Konfiguration besitzt, die Carboxylgruppe exo- oder endo orientiert ist und die chiralen C-Atome, die mit einem Stern (*) gekennzeichnet sind, und C-Atom 2 die S-Konfiguration besitzen.

Die Erfindung betrifft ferner Verfahren zur Herstellung der Verbindungen der Formel I. Eine Verfahrensvariante ist dadurch gekennzeichnet, daß man eine Verbindung der Formel II,

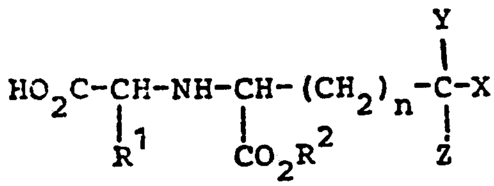

(II)

worin n $R^1$, $R^2$, X, Y und Z die Bedeutungen wie in Formel I haben, mit einer Verbindung der Formel III,

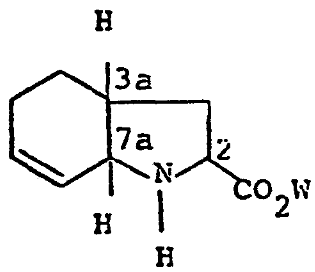

III

in welcher

W = Wasserstoff oder einen sauer abspaltbaren Rest, insbesondere einen tert.-Butyl-Rest bedeuten,

nach bekannten Amidbildungsmethoden der Peptidchemie umsetzt und gegebenenfalls anschließend durch Säurebehandlung den Rest W und gegebenenfalls durch zusätzliche Säure- oder Basenbehandlung auch den Rest $R^2$ abspaltet, wobei jeweils die freien Carbonsäuren erhalten werden.

Weitere Syntheseverfahren zur Herstellung der Verbindungen der Formel I, in der Y und Z zusammen Sauerstoff bedeuten, bestehen darin, daß man eine Verbindung der Formel IV,

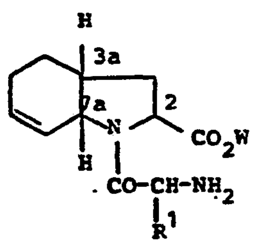

(IV)

in welcher $R^1$ die Bedeutung wie in Formel I und W die Bedeutung wie in Formel III besitzen, mit einer Verbindung der Formel V

$R^2O_2C\text{-}CH=CH\text{-}CO\text{-}X$ (V)

worin $R^2$ und X die Bedeutungen wie in Formel I besitzen, in bekannter Weise in einer Michael-Reaktion (Organikum, 6. Auflage, S. 492, 1967) umsetzt und gegebenenfalls den Rest W und/oder den Rest $R^2$, wie oben beschrieben, abspaltet oder daß man eine Verbindung der obengenannten Formel IV mit einer Verbindung der allgemeinen Formel VI, worin $R^2$ die Bedeutung wie in Formel I hat, und mit einer Verbindung der allgemeinen Formel VII

$OHC\text{-}CO_2R^2$ $X\text{-}CO\text{-}CH_3$

(VI) (VII)

worin X die Bedeutung wie in Formel I hat, in bekannter Weise in einer Mannich-Reaktion (Bull. Soc. Chim. France 1973, S. 625) umsetzt und anschließend gegebenenfalls den Rest W und/oder den Rest $R_2$ wie oben beschrieben unter Bildung der freien Carboxygruppen abspaltet.

Ferner können Verbindungen der Formel I mit Y und Z jeweils = Wasserstoff auch in der Weise hergestellt werden, daß man eine Verbindung der oben genannten Formel IV gemäß der in J. Amer. Chem. Soc. 93, 2897 (1971) beschriebenen Verfahrensweise mit einer Verbindung der Formel VIII

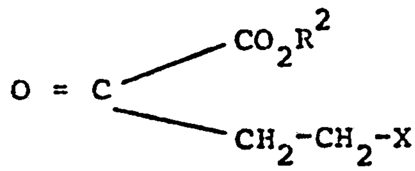

(VIII)

worin $R^2$ und X die Bedeutungen wie in Formel I besitzen, umsetzt, die erhaltenen Schiff-Basen reduziert und anschließend gegebenenfalls den Rest W und/oder den Rest $R^2$, wie oben beschrieben, unter Bildung der freien Carboxygruppen abspaltet. Die Reduktion der Schiff-Basen kann elektrolytisch oder mit Reduktionsmitteln wie beispielsweise Natriumborhydrid oder Natriumcyanborhydrid erfolgen.

Verbindungen der Formel I mit Y = Hydroxy und Z = Wasserstoff können beispielsweise auch durch Reduktion einer gemäß obigen Verfahrensweisen erhaltenen Verbindung I mit Y und Z = zusammen Sauerstoff erhalten werden. Diese Reduktion kann mit einem Reduktionsmittel wie Natriumborhydrid und anderen komplexen Boranaten oder beispielsweise Boran-Amin-Komplexen, erfolgen.

Verbindungen der Formel I, in welcher R für Wasserstoff steht, können gegebenenfalls nach an sich bekannten Methoden in ihre Ester der Formel I, worin R ($C_1$ bis $C_6$)-Alkyl oder ($C_7$-$C_9$)-Aralkyl bedeutet, überführt werden.

Die Erfindung betrifft auch Verbindungen der Formel III,

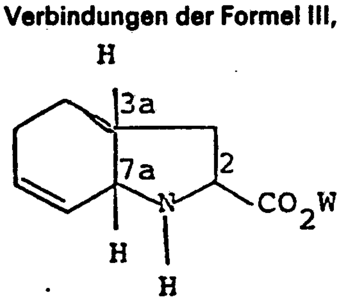

(III)

in der die H-Atome an den C-Atomen 3 a und 7 a zueinander cis-konfiguriert sind und die Gruppe $-CO_2W$ an C-Atom 2 exo- oder endo-ständig zum bicyclischen Ringsystem orientiert ist und worin

W = Wasserstoff oder einen sauer abspaltbaren Rest bedeutet.

Diese Verbindungen dienen gemäß der Erfindung als Ausgangsstoffe bei der Synthese von Verbindungen der Formel I und können erfindungsgemäß nach folgender Verfahrensweise hergestellt werden:

Verbindungen der Formel IX,

(IX)

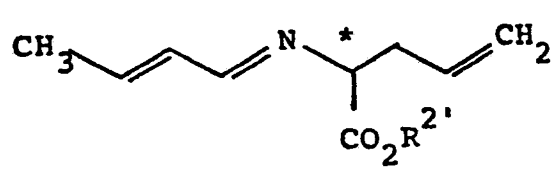

in welcher $R^{2'}$ ($C_1$ bis $C_6$)-Alkyl oder ($C_7$ bis $C_8$)-Aralkyl bedeutet, werden mit Acylierungsmitteln, die die Gruppe $-CO-R^3$, worin der Rest $R^3$ für ($C_1$ bis $C_6$)-Alkyl, ($C_5$ bis $C_9$)-Cycloalkyl, ($C_2$ bis $C_6$)-Alkenyl, ($C_1$ bis $C_6$)-Alkoxy, Aryl, Aryloxy, Aryl-($C_1$ bis $C_4$)-alkyl oder Aryl-($C_1$ bis $C_4$)-alkoxy steht, übertragen, zu Verbindungen der Formel X,

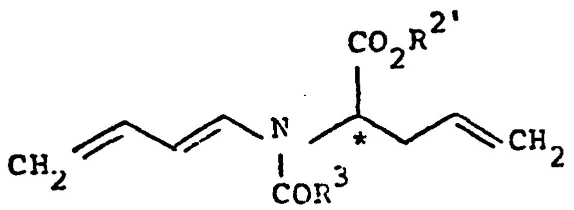

(X)

in welcher $R^{2'}$ und $R^3$ vorstehende Bedeutung haben, umgesetzt,

diese zu einem Gemisch stereoisomerer Verbindungen der Formel XI,

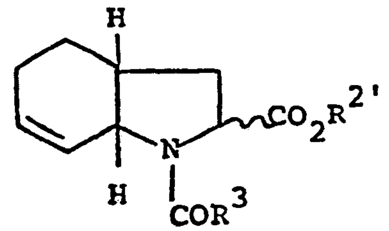

(XI)

in welcher $R^{2'}$ und $R^3$ vorstehende Bedeutung haben und die Wasserstoffatome an dem Brückenkopf-C-Atomen ciskonfiguriert sind, cyclisiert,

dieses Stereoisomerengemisch, in dem je nachdem, ob von R-, S-konfigurierten oder racemischen Verbindungen der Formel IX ausgegangen wurde, Diastereomerenpaare der Formeln XI b und d bzw. XI a und c, bzw. Gemische der Formeln XI a - d,

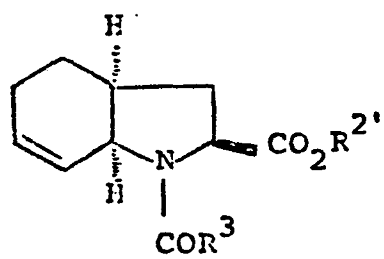

(XI a)

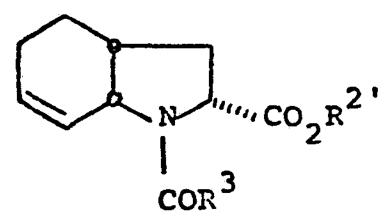

(XI b)

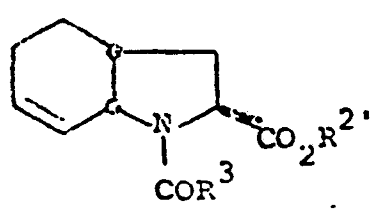

(XI c)

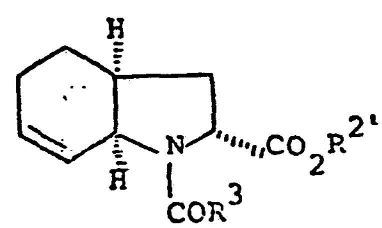

(XI d)

in welchen $R^{2'}$ und $R^3$ jeweils die vorstehende Bedeutung haben, vorliegen, gegebenenfalls nach vorheriger Auftrennung in Enantiomere, Diastereomerenpaare oder Racemate, zu Verbindungen der Formel III, in welcher W, für Wasserstoff steht, verseift und

diese gegebenenfalls zu Verbindungen der Formel III, in welcher W für einen sauer abspaltbaren Rest steht, verestert.

Bei der Synthese von Verbindungen der Formel IX geht man von einem Allylglycinester der Formel XII aus,

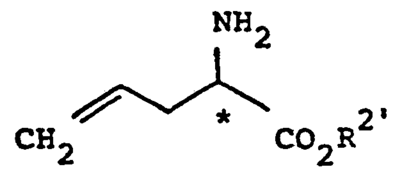

(XII)

wobei $R^{2'}$ ($C_1$ bis $C_6$)-Alkyl oder ($C_7$ oder $C_8$)-Aralkyl bevorzugt ($C_1$ bis $C_4$)-Alkyl oder Benzyl ist. Der Allylglycinester kann als racemische Verbindung oder als optisch reine R- oder S-Form in die Synthese eingesetzt werden. Die Synthese des racemischen Allylglycins ist in Monatshefte der Chemie 85, 1071 (1954) beschrieben; die der S- und R-Verbindungen im J. biol. Chem. 223, S. 40 (1955). Die Veresterung erfolgt nach bekannten Methoden der organischen Chemie. Die Herstellung des Ethylesters ist im Monatshefte-der-Chemie-Artikel beschrieben.

Die Verbindung der Formel XII wird mit Crotonaldehyd in einem organischen Lösungsmittel unter Wasserabscheidung zur Schiffbase der Formel IX umgesetzt. Die Reaktion wird bei Temperaturen im Bereich von -40° bis +80° C, vorzugsweise bei +20° C durchgeführt. Es werden aprotische Lösungsmittel, wie z.B. Benzol, Toluol, Tetrachlorkohlenstoff oder Chloroform, verwendet. Das Reaktionswasser wird mit einem Wsserbindenden Mittel, wie z.B. $MgSO_4$, $Na_2SO_4$, Molekularsieb oder einem organischen Hilfsstoff, wie z.B. Orthoameisensäureester oder mittels azeotroper Wasserabscheidung aus dem Reaktionsgleichgewicht entfernt.

Die Schiffbase wird vorzugsweise in einen aprotischen Lösungsmittel, wie z.B. Methylenchlorid, Chloroform, Toluol oder Dimethylformamid, in Gegenwart einer Base wie z.B. Triäthylamin, Collidin, Pyridin oder andere Amine, mit einem Acylierungsmittel, wie z.B. einem Säurechlorid zu dem Dienamid der Formel X acyliert. Die Reaktion wird bei Temperaturen im Bereich von -80° bis +40° C durchgeführt. Vorzugsweise wird die Reaktion bei -80° C begonnen und bei 20° C zu Ende geführt.

In dem die Gruppe -CO-$R^3$ übertragenden Acylierungsmittel bedeutet $R^3$ vorzugsweise Phenyl, tert.-Butoxy, Methyl, Methoxy oder Äthoxy. Die Verbindung der Formel X wird vorzugsweise in einem hochsiedenden organischen Lösungsmittel erhitzt und in einer intramolekularen Diels-Alder-Reaktion zu den bicyclischen Aminosäurederivaten XI a - d, cyclisiert, die als Stereoisomerengemisch anfallen.

Als Lösungsmittel können solche zur Verwendung kommen, die in einem Temperaturbereich zwischen 80 und 250° C sieden, wic z.B. Toluol, Xylol, Dichlorbenzol. Die Thermolyse ist auch mit einem tiefer siedenden Lösungsmittel in einer Druckapparatur möglich. Verzugsweise wird die Thermolyse in einem Temperaturbereich zwischen 100 und 180° C bei Normaldruck durchgeführt. Zur Desaktivierung der Glasoberfläche des Reaktionsgefäßes werden gegebenenfalls Bistrimethylsilylacetamid oder übliche desaktivierende Verbindungen bzw. Säurefänger und ein Radikalfänger wie z.B. tert.-Butylcatechol zur Unterdrückung radikalischer Nebenreaktionen zugegeben.

Die endo-cis-Verbindungen XI a und XI b und die exo-cis-Verbindungen XI c und XI d liegen jeweils als Racemate vor bei Verwendung des racemischen Allylglycinesters XII. Bei Einsatz des S-konfigurierten Allylglycinesters als Ausgangsverbindung erhält man die Aminosäurederivate XI a mit der cis-endo-S-Konfiguration und XI c mit der cis-exo-Konfiguration, wobei die S-Konfiguration sich auf das C-Atom 2 bezieht. Bei Verwendung des R-konfigurierten Allylglycinesters erhält man entsprechend die am Kohlenstoff 2 R-konfigurierten Aminosäurederivate XI b und XI d. Die Racemate XI a/XI b und XI c/XI d bzw. die Diasteromeren XI a und XI c bzw. XI b und XI d lassen sich beispielsweise durch fraktionierte Kristallisation oder durch Säulenchromatographie an Kieselgel nach üblichen Methoden trennen. In die weiteren Reaktionen können die Racemate oder die optisch reinen Diasteromeren eingesetzt werden.

Durch saure oder alkalische Verseifung werden die Aminosäuren der Formel III erhalten, worin W Wasserstoff bedeutet. Die Aminosäuren können gegebenenfalls veresteit werden. Die bevorzugten tert.-Butylester der Aminosäuren der Formel III (W = tert.-Butyl) werden nach den in der Peptidchemie üblichen Methoden erhalten, wie z.B. durch Reaktion der Säuren mit Isobutylen in einem inerten organischen Lösungsmittel (z.B. Dioxan) in Gegenwart von Säuren (wie z.B. Schwefelsäure). Als besonders vorteilhaft hat sich das folgende Verfahren erwiesen: Die entsprechende Aminosäure wird mit einer basisch abspaltbaren Gruppe, wie z.B. der Methylsulfonylethoxycarbonylgruppe (= MSC), Tesser, Balvert-Geers, Int. J. Pept. Protein Res 7, 295 (1975) am Stickstoff acyliert.

Die Carbonsäure wird im neutralen bis schwach basischen pH-Bereich mit tert.-Butanol in einem organischen Lösungsmittel, wie z.B. Pyridin, in Gegenwart von Propylphosphonsäureanhydrid zum entsprechenden tert.-Butylester umgesetzt. Durch Abspaltung der MSC-Schutzgruppe im stark alkalischen pH-Bereich mit Alkali im wässrigen Lösungsmittel wird der tert.-Butylester der Formel III erhalten (W = tert.-Butyl).

Die als Ausgangsstoffe zur Herstellung der Verbindungen der Formel 1 verwendeten Verbindungen der Formel II mit n = 1, Y, Z = Wasserstoff, $R^1$ = Methyl und $R^2$ = Methyl oder Ethyl und X = Phenyl sind bekannt (europäische Patentanmeldung Nr. 37 231). Die Verbindungen der Formel II lassen sich nach verschiedenen Verfahrensweisen herstellen. Eine Synthesevariante geht von einem Keton der oben genannten Formel VII aus, das nach bekannten Verfahrensweisen in einer Mannich-Reaktion mit einer Verbindung der

oben genannten Formel VI zusammen mit Aminosäureestern der Formel XIII

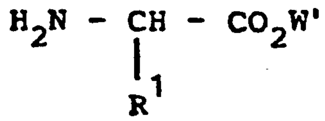

(XIII)

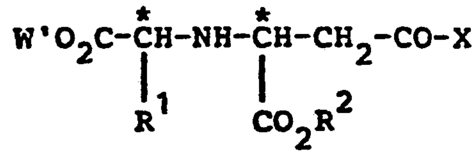

(XIV)

worin $R^1$ die oben genannte Bedeutung besitzt und W' einen hydrogenolytisch oder sauer abspaltbaren Rest, insbesondere einen Benzyl- oder einen tert.-Butyl-Rest bedeutet, zu einer Verbindung der Formel XIV, worin $R^2$, $R^2$, X und W' die oben genannten Bedeutungen besitzen, mit der Einschränkung, daß, wenn W' einen hydrogenolytisch abspaltbaren Rest, insbesondere Benzyl, bedeutet, $R^2$ nicht die Bedeutung von W' besitzen darf, umsetzt. Spaltet man den Rest W' hydrogenolytisch mit Hilfe von beispielsweise Palladium ab, werden bei einer Wasserstoffaufnahme von 3 Moläquivalenten Verbindungen der Formel II mit Y, Z = Wasserstoff erhalten. Stoppt man die Wasserstoffaufnahme bei 1 Moläquivalent, erhält man Verbindungen dei Formel II mit n = 1 und Y und Z zusammen = Sauerstoff, die man ebenfalls erhält, wenn der Rest W' der Formel XIV mit Säuren, wie beispielsweise Trifluoressigsäure oder Salzsäure, in einem inerten organischen Lösungsmittel, wie beispielsweise Dioxan, abgespalten wird.

Verbindungen der Formel XIV sind auch durch Michael-Additionen einer Verbindung der oben genannten Formel V mit einer Verbindung der oben genannten Formel XIII nach bekannten Verfahrensweisen zugänglich. Bevorzugt eignet sich dieses Verfahren zur Herstellung von solchen Verbindungen der Formel XIV, in denen $R^1$ = Methyl, $R^2$ = Ethyl und X = Aryl bedeuten.

Die Verbindungen der Formel XIV fallen als Diastereomerengemische an. Bevorzugte Diastereomeren der Formel XIV sind solche, in denen die mit einem Stern markierten chiralen C-Atome jeweils S-Konfiguration aufweisen. Diese können beispielsweise durch Kristallisation oder durch Chromatographie, z.B. an Kieselgel, abgetrennt werden. Bei der nachfolgenden Abspaltung des Restes W' bleiben die Konfigurationen der chiralen C-Atome erhalten.

Die als Ausgangsstoffe zur Herstellung der Verbindungen der Formel I verwendeten Verbindungen der oben genannten Formel IV werden aus den Verbindungen der oben genannten Formel III durch Umsetzen mit einer N-geschützten 2-Aminocarbonsäure der Formel XV

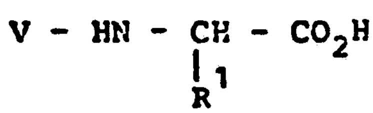

(XV)

worin V eine Schutzgruppe und $R^1$ die oben genannte Bedeutung besitzt, nach bekannten Verfahrensweisen erhalten. Als Schutzgruppe V, die nach beendeter Reaktion wieder abgespalten wird, kommt beispielsweise tert.-Butoxycarbonyl in Frage.

Die Umsetzung einer Verbindung der Formel II mit einer Verbindung der Formel III zur Herstellung einer Verbindung der Formel I erfolgt gemäß einer in der Peptidchemie bekannten Kondensationsreaktion, wobei als Kondensationsmittel beispielsweise Dicyclohexylcarbodiimid und 1-Hydroxy-benzotriazol zugesetzt werden. Bei der nachfolgenden sauren Abspaltung des Restes W werden als Säuren bevorzugt Trifluoressigsäure oder Chlorwasserstoff eingesetzt.

In den oben beschriebenen Reaktionen zur Herstellung der Verbindungen der Formeln III, IV und I bleiben jeweils die Konfigurationen der Zwischenprodukte an den Brückenkopf C-Atomen 3 a und 7 a erhalten.

Die gemäß oben beschriebener Verfahrensweise erhaltenen Verbindugen der Formel III fallen als Gemisch an und können beispielsweise durch Umkristallisation oder durch Chromatographie voneinander getrennt werden.

Die Verbindungen der Formel III fallen als racemische Gemische an und können als solche in die weiteren oben beschriebenen Synthesen eingesetzt werden. Sie können aber auch nach Auftrennung der Racemate mit üblichen Methoden, beispielsweise über Salzbildung mit optisch aktiven Basen oder Säuren in die optischen Antipoden als reine Enantiomere eingesetzt werden. Die reinen Enantiomeren können auch erhalten werden.

Fallen die Verbindungen der Formel I als Racemate an, können auch diese nach den üblichen Methoden wie beispielsweise über Salzbildung mit optisch aktiven Basen oder Säuren in ihre Enantiomeren gespalten oder durch Chromatographie getrennt werden.

Die erfindungsgemäßen Verbindungen der Formel I, liegen, falls R = Wasserstoff ist als innere Salze vor. Als amphotere Verbindungen können sie Salze mit Säuren oder Basen bilden. Diese Salze werden in üblicher Weise durch Umsetzen mit einem äquivalent Säure bzw. Base hergestellt.

Die Verbindungen der Formel I und deren Salze besitzen lang andauernde, intensive blutdrucksenkende Wirkung. Sie sind starke Hemmer des Angiotensin-Converting-Enzyms (ACE-Hemmer). Sie können zur

Bekämpfung des Bluthochdrucks verschiedener Genese eingesetzt werden. Auch ihre Kombination mit anderen blutdrucksenkenden, gefäßerweiternden oder diuretisch wirksamen Verbindungen ist möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1-100 mg, vorzugsweise bei 1-40 mg je Einzeldosis bei einem normalgewichtigen erwachsen Patienten; dies entspricht einer Dosis von 0,013-1,3 mg/kg/Tag, vorzugsweise 0,013 - 0,53 mg/kg/Tag. Sie kann in schweren Fällen auch erhöht werden, da toxische Eigenschaften bisher nicht beobachtet wurden. Auch eine Herabsetzung der Dosis ist möglich und vor allem angebracht, wenn gleichzeitig Diuretika verabreicht werden.

Die erfindungsgemäßen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vemischt und durch übliche Methoden in geeignete Darreichungsformen gebracht, wie Tabletten, Dragees, Steckkapseln, wäßrige, alkoholische oder ölige Suspensionen oder wäßrige, alkoholische oder ölige Lösungen. Als inerte Träger können z.B. Gummi arabicum, Magnesiumkarbonat, Kaliumphosphat, Milchzucker, Glucose oder Stärke, insbesondere Maisstarke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspensionen oder Emulsionen gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. äthanol, Propandiol oder Glycerin, daneben auch Zuckerlösungen wie Glukose- oder Mannitlösungen, oder auch eine Mischung aus den verschiedenen genannten Lösungsmitteln.

Die außerordentlich starke Wirksamkeit der Verbindungen gemäß Formel I wird durch die pharmakologischen Daten der nachfolgenden Tabellen belegt:

Intraduodenale Gabe an der narkotisierten Ratte, 50 % Hemmung der durch 310 ng Angiotensin I ausgelösten Presserreaktion 30 min. nach Applikation in der Dosis ........ = $ED_{50}$:

**Tabelle I**

(H-Atome an C-3a und C-7a in Formel I besitzen cis-Konfiguration die Carboxylgruppe an C-2 die endo-Konfiguration und die mit * bezeichneten C-Atome und C-2 die S-Konfiguration, wenn keine anderen Angaben in der Tabelle gemacht sind)

| n | X | Y | Z | $R^2$ | $R^1$ | R | $ED_{50}$ (µg/kg) |
|---|---|---|---|---|---|---|---|
| 1 | 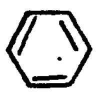 | H | H | $C_2H_5$ | $CH_3$ | H | 40 |
| 1 | 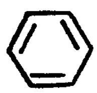 | H | H | H | $CH_3$ | H | 600 |
| 1 | 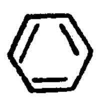 | H | H | $C_2H_5$ | $CH_3$ | H | 80 (R, S-Konfiguration an C-2 der Formel I) |
| 1 | 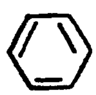 | – O – | | $C_2H_5$ | $CH_3$ | H | 390 |

Die Symbole n, X, Y, Z, R, $R^1$ und $R^2$ beziehen sich auf die Verbindungen der Formel I.

**Tabelle II**

(H-Atome an C-3a und C-7a in Formel 1 besitzen cis-Konfiguration, die Carboxylgruppe die exo-Konfiguration und die mit * bezeichneten C-Atome und C-2 die S-Konfiguration)

| n | X | Y | Z | $R^2$ | $R^1$ | R | $ED_{50}$ (µg/kg) |
|---|---|---|---|---|---|---|---|
| 1 | 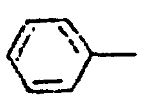 | H | H | $C_2H_5$ | $CH_3$ | H | 70 |
| 1 | 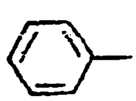 | H | H | H | $CH_3$ | H | 700 |
| 1 | 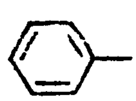 | – O – | | $C_2H_5$ | $CH_3$ | H | 500 |

Die Symbole n, X, Y, Z, R, $R^1$ und $R^2$ beziehen sich Verbindungen der Formel I.

Die nachfolgenden Beispiele dienen zur Erläuterung der Erfindung, ohne diese auf die stellvertretend genannten Verbindungen zu beschränken.

Die in den folgenden Beispielen angegebenen $^1$H-NMR-Daten wurden durch Messung in $CDCl_3$ ermittelt und sind in δ (ppm) angegeben.

**Beispiel 1**

N-(1-S-Carbethoxy-3-phenyl-propyl)-S-alanyl-cis-2,3,3a, 4,5,7a-hexahydro/IH/indol-2-R,S-endo-carbonsäure

a) N-Benzoyl-N-(butadien-1,3-yl)-R,S-allylglycinethylester

Man löst 28,6 g R,S-Allylglycinethylester in 140 ml Toluol und gibt 14,0 g Crotonaldehyd zu. Nach Zugabe von 30 g wasserfreiem Magnesiumsulfat wird 3 Stunden bei Raumtemperatur gerührt. Danach wird vom Magnesiumsulfat abgesaugt und das Toluol im Vakuum abgezogen. Rückstand: 44,4 g öl.

Man kühlt 750 ml Methylenchlorid auf -70°C ab und gibt 44,4 g Triethylamin sowie 28,1 g Benzoylchlorid zu. Dazu werden die oben erhaltenen 44,4 g der Schiffbase gefügt. Es wird 2 Stunden bei -75°C gerührt. Anschließend läßt man auf 0°C erwärmen. Das Lösungsmittel wird bei Raumtemperatur abrotiert. Nun nimmt man den Rückstand in Toluol auf, wäscht mit Wasser, trocknet und rotiert das Toluol ab. Der Rückstand wird über eine kurze Säule mit Kieselgel (500 ml) filtriert. Das Elutionsmittel ist dabei Methylenchlorid. Nach dem Abdampfen des Methylenchlorids erhält man 49 g N-(Benzoyl)-N-(butadien-1,3-yl)-R,S-allylglycinethyl-ester.

$^1$H-NMR-Daten:
1,1 - 1,4 (t, 3H),
2,7 - 3,1 (m, 2H),
4,0 - 4,5 (m, 3H),
4,8 - 6,8 (m, 8H),
7,3 - 7,6 (s, 5H).

b) N-Benzoyl-cis-2,3,3a,4,5,7a-hexahydro/IH/indol-2-R,S-endo-carbonsäureethylester

49 g N-(Benzoyl):N-(butadien-1,3-yl)-R,S-allylglycin-ethyl-ester werden in 1000 ml Xylol gelöst und mit 0,1 g tert.-Butylcatechol sowie 5 Tropfen Bistrimethylsilylacetamid versetzt. Es wird 6 Stunden unter Stickstoff zum Rückfluß erhitzt, wobei nach 3 Stunden weitere 0,1 g tert.-Butylcatechol sowie 5 Tropfen Bistrimethylsilylacetamid zugegeben werden. Es wird von Xylol abrotiert, wobei 43 g öliger Rückstand zurückbleibt.

70 g öl werden an Kieselgel mit Toluol/Essigester 4:1 chromatographiert. Es werden 53 g N-Benzoyl-cis-2,3, 3a,4,5,7a-hexahydro/1H/indol-2-R,S-endo-carbonsäure-ethylester (Rf: 0,18) als öl und 7,5 g N-Benzoyl-cis-2,3,3a,4,5,7a-hexahydro/1H/indol-2-R,S-exo-carbonsäure-ethylester (Rf: 0,26, Fp.= 96-98°C) erhalten. Beide Verbindungen liegen als Racemat vor.

c) cis-2,3,3a,4,5,7a-hexahydro/1H/indol-2-R,S-endo-carbon-säure

1,5 g N-Benzoyl-cis-2,3,3a,4,5,7a-hexahydro/1H/indol-2-endo-R,S-carbonsäureethylester werden in 10 ml Ethanol gelöst. Dazu gibt man 0,35 g Kaliumhydroxid gelöst in 5 ml Wasser. Es wird 2 1/2 Stunden bei Zimmertemperatur gerührt, mit Wasser verdünnt und mit Essigester extrahiert. Die wäßrige Lösung wird mit 2n Salzsäure angesäuert und nochmals mit Essigester extrahiert. Nach dem Trocknen und Einrotieren erhält man 0,9 g N-Benzoyl-cis-2,3,3a,4,5,7a-hexahydro[1H]indol-2-endo-carbonsäure vom Fp: 153 - 154°C. 13,4 g der N-Benzoyl-carbonsäure werden in einem Gemisch von 200 ml 2n Salzsäure und 67 ml Ethanol gelöst und 22 Stunden bei einer Badtemperatur vcn 110°C unter Stickstoff erhitzt. Der Alkohol wird im Vakuum abgezogen und die wäßrige Phase mit Methylenchlorid extrahiert. Danach wird mit konz. Natronlauge auf pH 7,0 gebracht und im Vakuum zur Trockene eingedampft. Der Rückstand wird in Methylenchlorid/Methanol aufgenommen, vom Salz abfiltriert, eingeengt und aus Ethanol/Ether umkristallisiert. Ausbeute: 7.0 g, Fp: 220°C (Zers.)

d) N-(1-S-Carbethoxy-3-phenyl-propyl)-S-alanyl-cis-2,3, 3a,4,5,7a-hexahydro/1H/indol-2-R,S,-endo-carbonsäure

0,84 g N-(1-S-Carbethoxy-3-phenyl-propyl)-S-alanin, 0,69 g N-Hydroxysuccinimid sowie 0,66 g Dicyclohexylcarbo-diimid werden in 8 ml trockenem Dimethylformamid bei 0°C gelöst. Anschließend wird 3 Stunden bei Raumtemperatur gerührt. Nun setzt man eine Lösung von 0,5 g cis-2,3,3a,4,5,7a-hexahydro[1H]indol-2-R,S-endo-carbonsäure in 10 ml Dimethylformamid und 10 ml Wasser in Gegenwart von 0.27 g konz. Natronlauge zu. Es wird 12 Stunden bei Raumtemperatur gerührt, vom gebildeten Dicyclohexylharnstoff abgesaugt und mit 2n Salzsäure neutral gestellt. Dann wird im Vakuum zur Trockene eingeengt, in Essigester aufgenommen, vom Ungelösten abfiltriert und die Essigesterlösung im Vakuum eingeengt. Der Rückstand wird über Kieselgel mit Methylenchlorid/Methanol 9:1 als Eluierungsmittel gereinigt. Ausbeute: 0.13 g (Rf: 0.74).

**Beispiel 2**

N-(1-S-Carbethoxy-3-phenyl-propyl)-S-alanyl-cis-2,3,3a, 4,5,7a-hexahydro[1H]indol-2-S-endo-carbonsäure

Man erhält die Verbindung, indem man das Diastereomerengemisch aus Beispiel 1 d über ausreichend Kieselgel mit Methylenchlorid/Methanol 9:1 als Elutionsmittel trennt. $R_f$; 0,65.

**Beispiel 3**

N-(1-S-Carbethoxy-3-phenyl-propyl)-S-alanyl-cis-2,3,3a,4,5,7a-hexahydro[1H]indol-2-R-endo-carbonsäure

Man erhält die Verbindung, indem man das Diastereomerengemisch aus Beispiel 1 d über ausreichend Kieselgel mit Methylenchlorid/Methanol 9:1 als Elutionsmittel trennt. $R_f$ = 0,8.

**Beispiel 4**

cis-2,3,3a,4,5,7a-hexahydro[1H]indol-2-R,S-endo-carbon-säure-tert.butylester

Man löst 3,35 g cis-2,3,3a,4,5,7a-hexahydro[1H]indol-2-R,S-endo-carbonsäure in 50 ml Dimethylformamid und gibt 5,3 g Methylsulfonylethylsuccinimidecarbonat und 2.3 g Ethylmorpholin zu. Man läßt 12 Stunden bei Raumtemperatur stehen, dampft im Vakuum das Lösungsmittel ab und löst den Rückstand in Essigester. Die Essigesterlösung wird mit gesättigter Natriumbicarbonatlösung ausgeschüttelt, mit 2n Chlorwasserstoffsäure auf pH 3,5 gebracht und mit Essigester mehrere Male extrahiert. Die vereinigten Essigesterlösungen werden nach dem Trocknen eingeengt. Man erhält als Rückstand 8,5 g N-Methylsulfonylethyl-oxycarbonyl-cis-2,3,3a,4,5,7a-hexahydro[1H]indol-2-R,S-endo-carbonsäure, der in 10 ml Pyridin gelöst wird. Dazu werden bei 0°C 10 ml tert.-Butanol und 12 ml einer 50 %igen Lösung Propylphosphonsäureanhydrid in Methylenchlorid gegeben. Es wird 6 Stunden bei 40°C gerührt, 200 ml Essigester zugegeben mit Kaliumhydrogensulfatlösung versetzt, bis ein pH von 3,5 erreicht ist. Es wird mit Essigester extrahiert, die Essigesterlösung mit Wasser gewaschen, getrocknet und im Vakuum eingeengt. Das zurückbleibende öl wird über Kieselgel mit Essigester als Elutionsmittel gereinigt. Der tert.-Butylester wird als öl erhalten.

$^1$H-NMR-Daten:
1,4 (s, 9H),
1,0 - 2,8 (m, IH)
2,9 (s, 3H)
3,1 - 3,5 (m, 2H)
3,7 - 4,8 (m, 4H)
5,6 - 5,9 (m, 2H).

1,35 g tert.-Butylester werden in 15 ml Methanol und 1,5 ml Wasser gelöst. Es wird mit 2n Natronlauge auf pH 13 gebracht und 2 Stunden bei Raumtemperatur gerührt. Danach wird mit 2 n Salzsäure neutralisiert, Methanol im Vakuum abgedampft, die wäßrige Phase mit Essigester extrahiert, die Essigesterlösung mit Wasser gewaschen, getrocknet und eingeengt. Der ölige Rückstand wird über Kieselgel mit Essigester als Elutionsmittel gereinigt.

Man erhält so 0,3 g cis-2,3,3a,4,5,7a-hexahydro[1H]indol-2-R,S-endo-carbonsäure-tert.-butylester als öl

$^1$H-NMR-Daten:
1,4 (s, 9H)
1,0 - 2,7 (m, 8H)
3,2 - 4,9 (m, 2H)
5,8 (d, 2H).

**Beispiel 5**

N-(1-S-Carbethoxy-3-phenyl-propyl)-S-alanyl-cis-2,3,3a,4 5,7a-hexahydro[1H]indol-2-S-endo-carbonsäure-tert.-butylester

0,28 g N-(1-S-Carbethoxy-3-phenyl-propyl)-S-alanin werden in 4 ml Dimethylformamid gelöst. Nun werden bei Raumtemperatur 0,15 g Hydroxybenzotriazol und 0,22 g Dicyclohexylcarbodiimid zugegeben. Es wird 4 Stunden bei Raumtemperatur gerührt. Anschließend werden 0.24 g tert.-Butylester aus Beispiel 4 zugegeben und 20 Stunden bei Raumtemperatur gerührt. Man verdünnt mit Essigester saugt vom Harnstoff ab und dampft im Vakuum ein. Der Rückstand wird in Essigester aufgenommen, die Essigesterlösung mit Bicarbonatlösung gewaschen, getrocknet und eingeengt. Der ölige Rückstand (0,6 g) wird über Kieselgel mit Essigester/Cyclohexan 2:1 als Elutionsmittel in die Diastereomeren getrennt.

Man erhält 0,24 g des tert. Butylesters mit der 2-S-endo-Konfiguration. ($R_f$: 0.45, m/e: 484)

$^1$H-NMR-Daten:
1,4 (s, 9H)
0,8 - 3,8 (m, 20H)
3,9 - 4,6 (m, 4H)
5,4 - 6,0 (m, 2H)
7,2 (s, 5H).

**Beispiel 6**

N-(1-S-Carbethoxy-3-phenyl-propyl)-S-alanyl-cis-2,3,3a, 4,5,7a-hexahydro[1H]indol-2-R-endo-carbonsäure-tert.butylester

Dieser Ester wird bei der Chromatographie des Diastereomerengemisches aus Beispiel 5 an Kieselgel, wie in Beispiel 5 beschrieben, erhalten. Elutionsmittel ist Essigester/Cyclohexan. Ausbeute: 0.16 g ($R_f$: 0.3; m/e 484).

**Beispiel 7**

N-(1-S-Carbethoxy-3-phenyl-propyl)-S-alanyl-cis-2,3,3a,4, 5,7a-hexahydro[1H]indol-2-R-endo-carbonsäuretrifluoracetat

0,16 g des tert.-Butylesters, der in Beispiel 6 erhalten wurde, weiden in 1 ml Trifluoressigsäure bei 0°C gelöst und bei dieser Temperatur 3 Stunden gerührt. Die Trifluoressigsaure wird im Vakuum abgedampft und der Rückstand aus Diisopropylether kristallisiert.

Ausbeute an Trifluoracetat: 0,08 g vom Fp: 120 - 121°C. Das Trifluoracetat kann mit basischen Ionenaustauschern (OH -Form) in Methanol/Wasser 60:40 in die Aminosäure überführt werden.

**Beispiel 8**

N-(1-S-Carbethoxy-3-phenyl-propyl)-S-alanyl-cis-2,3,3a, 4,5,7a-hexahydro[1H]indol-2-S-endo-carbonsäuretrifluoracetat

0,24 g des tert.-Butylesters, der in Beispiel 5 beschrieben ist, werden in 1,5 ml Trifluoressigsäure bei 0°C gelöst und bei dieser Temperatur 5 Stunden gerührt. Danach wird die Trifiuoressigsäure im Vakuum abgezogen und der Rückstand aus Diisopropylether/Petrolether kristallisiert. Ausbeute: 0,1g; m/e (nach Silylierung) 500

**Beispiel 9**

N-(1-S-Carbethoxy-3-phenyl-propyl)-S-alanyl-cis-2,3,3a, 4,5,7a-hexahydro[1H]indol-2-S-endo-carbonsäurehydrochlorid

Eine Lösung von 0,6 g tert.-Butylester aus Beispiel 5 in 5 ml Methylenchlorid wird mit trockenem Chlorwasserstoffgas gesättigt und 16 Stunden bei 20 - 25°C stehengelassen. Die Lösung wird im Vakuum eingeengt. Der Rückstand wird mit Diisopropylether verrieben und abgesaugt.

Ausbeute 0.4 g

$^1$H-NMR-Daten: 0,9 - 3,0 (m, 17H)
3,4 - 4,9 (m, 6H)
5,2 - 6,0 (m, 2H)
7,2 (s, 5H).

Das Hydrochlorid kann auch durch Umsetzung des Trifluoracetats von Beispiel 8 mit schwach basischen Ionenaustauschern (Acetatform) auf pH 4,0 und anschließender Behandlung mit ethanolischer Salzsäure und Eindampfen und Verreiben mit Diisopropylether erhalten werden.

**Beispiel 10**

N-(1-S-Carboxy-3-phenyl-propyl)-S-alanyl-cis-2,3,3a, 4,5,7a-hexahydro[1H]indol-2-R,S-endo-carbonsäure

Eine lösung von 1 g N-(1-S-Carbethoxy-3-phenyl-propyl)-S-alanyl-cis-2,3,3a,4,5,7a-hexahydro[1H]indol-2-R,S-endo-carbonsäure in 10 ml Wasser wird mit einem äquivalent Kaliumhydroxid und einem 10 %igen Überschuß 4n Kaliumhydroxidlösung versetzt. Nach 8-stündigem Rühren bei 20 bis 25°C wird die Reaktionslösung mit 2n Salzsäure auf einen pH ± Wert von 4 gestellt und im Vakuum eingeengt. Man nimmt den Rückstand in Essigester auf und filtriert das abgeschiedene Salz ab. Die Essigesterlösung wird eingeengt, der Rückstand mit Diisopropylether verrieben und abgesaugt.

Ausbeute: 0,5 g

$^1$H-NMR-Daten: 1,5 (d, 3H)
(nach H/D-Austausch) 1,0 - 3,2 (m, 11H)
3,9 - 4,7 (m, 4H)
5,4 - 6,0 (m, 2H)
7,2 (s, 5H).

**Beispiel 11**

N-(1-S-Carboxy-3-phenyl-propyl)-S-alanyl-cis-2,3,3a 4,5,7a-hexahydro[1H]indol-2-S-endo-carbonsäure

Diese Verbindung wird aus der Verbindung von Beispiel 8 analog dem in Beispiel 10 beschriebenen Verfahren hergestellt.

$^1$H-NMR-Daten: 1,5 (d, 3H)
1,0 - 3,2 (m, 4H)
3,9 - 4,7 (m, 4H)
5,4 - 6,0 (m, 2H)
7,2 (s, 5H).

**Beispiel 12**

N-(1-S-Carbethoxy-3-phenyl-propyl)-S-alanyl-cis-2,3,3a-4,5,7a-hexahydro[1H]indol-2-R,S-exo-carbonsäure

a) cis-2,3,3a,4,5,7a-Hexahydro[1H]indol-R S-exo-carbonsäure

Diese Verbindung wird aus N-Benzoyl-cis-2,3,3a,4,5,7a-hexahydro[1H]indol-2-R,S-exo-carbonsäureethylester, der im Beispiel 1b beschrieben ist, analog dem in Beispiel 1c beschriebenen Verfahren hergestellt.

$^1$H-NMR-Daten: 1,6 - 2,5 (m, 3H)
(nach H/D-Austausch) 4,4 (s, 1H)
4,8 (m, 1H)
5,4 (m, 1H)
5,8 (m, 1H).

b) N-(1-S-Carbethoxy-3-phenyl-propyl)-S-alanyl-cis-2,3,3a-4,5,7a-hexahydro[1H]indol-2-R,S-exo-carbonsäure

Diese Verbindung wird aus der Verbindung von Beispiel 12 a und N-(1-S-Carbethoxy-3-phenyl-propyl)-S-alanin analog dem in Beispiel 1d beschriebenen Verfahren hergestellt.

$^1$H-NMR-Daten: 1,0 - 1,5 (m, 5H)
(nach H/D-Austausch) 1,6 - 3,4 (m, 13H)
3,5 - 4,8 (m, 5H)
5,2 - 6,0 (m, 2H)
7,2 (s, 5H).

**Beispiel 13**

N-(1-S-Carbethoxy-3-phenyl-propyl)-S-alanyl-cis-2,3,3a-4,5,7a-hexahydro[1H]indol-2-S-exo-carbonsäure

Diese Verbindung erhält man durch Säulenchromatographie des Diastereomerengemisches von Beispiel 12b an Kieselgel mit Methylenchlorid/Methanol 9=1 als Elutionsmittel ($R_f$: 0.6).

$^1$H-NMR-Daten: 1,0 - 1,5 (d,t 5H)
(nach H/D-Austausch) 1,6 - 3,5 (m, 13H)
3,5 - 4,8 (m, 5H)
5,3 - 6,0 (m, 2H)
7,2 (s, 5H).

**Beispiel 14**

cis-2-3,3a,4,5,7a-hexahydro[1H]indol-2-R,S-exo-carbon-säure-tert.-butylester

Diese Verbindung wird aus der Verbindung von Beispiel 12a analog dem in Beispiel 4 beschriebenen Verfahren hergestellt.

$^1$H-NMR-Daten: 1,1 - 2,8 (m, 8H)
1,4 (s, 9H)
3,3 - 4,9 (m, 2H)
5,8 (m, 2H).

**Beispiel 15**

N-(1-S-Carbethoxy-3-phenyl-propyl)-S-alanyl-cis-2,3,3a, 4,5,7a-hexahydro[1H]indol-2-S-exo-carbonsäure-tert.butylester

Diese Verbindung wird aus der Verbindung von Beispiel 14 und N-(1-S-Carbethoxy-3-phenyl-propyl)-S-alanin analog dem in Beispiel 5 beschriebenen Verfahren hergestellt. Das Diastereomerengemisch wird über Kieselgel mit Essigester/Cyclohexan 2:1 getrennt.
2-S-exo-tert.-Butylester ($R_f$: 0,5)
2-R-exo-tert.-Butylester ($R_f$: 0,4).

**Beispiel 16**

N-(1-S-Carbethoxy-3-phenyl-propyl)-S-alanyl-cis-2,3,3a, 4,5,7a-hexahydro[1H]indol-2-S-exo-carbonsäurehydrochlorid

Eine Lösung von 0,6 g tert.-Butylester aus Beispiel 15 in 5 ml Methylenchlorid wird mit trockenem Chlorwasserstoffgas gesättigt und 16 Stunden bei 20 - 25°C stehengelassen. Die Lösung wird im Vakuum eingeengt; der Rückstand wiid mit Diisopropylether verrieben und abgesaugt.
Ausbeute: 600 mg.
$^1$H-NMR-Daten: 0,8 - 3,1 (m, 17H)
3,5 - 4,8 (m, 6H)
5,3 - 6,0 (m, 2H)
7,2 (s, 5H).

**Beispiel 17**

N-(1-S-Carboxy-3-phenyl-propyl)-S-alanyl-cis-2,3,3a, 4,5,7a-hexahydro[1H]indol-2-S-carbonsäure

Diese Verbindung wird aus der Verbindung von Beispiel 16 analog dem in Beispiel 10 beschriebenen Verfahren hergestellt.
$^1$H-NMR-Daten: 1,5 (d, 3H)
0,9 - 3,4 (m, 11H)
3,9 - 4,8 (m, 4H)
5,2 - 5,9 (m, 2H)
7,2 (s, 5H).

**Beispiel 18**

N-(1-S-Carboxy-3-phenyl-propyl)-S-alanyl-cis-2,3,3a, 4,5,7a-hexahydro[1H]indol-2-R,S-exo-carbonsäure

Diese Verbindung wird aus der Verbindung von Beispiel 12 analog dem in Beispiel 10 beschriebenen Verfahren hergestellt.
$^1$H-NMR-Daten: 1,5 (d, 3H)
0,9 - 3,4 (m, 11H)
3,8 - 4,8 (m, 4H)
5,2 - 6,0 (m, 2H)
7,2 (s, 5H).

**Beispiel 19**

N-(1-S-Carbethoxy-3-phenyl-propyl)-S-alanyl-cis-2,3,3a, 4,5,7a-hexahydro[1H]indol-2-S-endo-carbonsäure

Diese Verbindung ist identisch mit der in Beispiel 2 beschriebenen Verbindung.

a) S-Allylglycinethylester

Die Herstellung erfolgt in der Weise wie sie in Monatshefte der Chemie 85, 1071 (1954) beschrieben ist.

b) N-(Benzoyl)-N-(butadien-1,3-yl)-S-allylglycinethylester

Diese Verbindung wird analog dem in Beispiel 1a beschriebenen Verfahren hergestellt.
$^1$H-NMR-Daten: 1,1 - 1,4 (tr, 3H)
2,7 - 3,1 (m, 2H)
4,0 - 4,5 (m, 3H)
4,8 - 6,8 (m, 8H)
7,3 - 7,6 (s, 5H)

c) N-Benzoyl-cis-2,3,3a,4,5,7a-hexahydro[1H]indol-2-S-endo-carbonsäureethylester und N-Benzoyl-cis-2,3,3a, 4,5,7a-hexahydro[1H]indol-2-S-exo-carbonsäureethylester

Dieses Diastereomerengemisch wird aus der Verbindung von Beispiel 19b analog dem in Beispiel 1b

beschriebenen Verfahren hergestellt. Das Gemisch wird über Kieselgel mit Toluol/Essigester 4:1 getrennt $R_f$-Wert der endo-Verbindung: 0,18; $R_f$-Wert der exo-Verbindung: 0,26.

$^1$H-NMR-Daten: 0,8 - 2,8 (m, 10H)
3,8 - 4,8 (m, 4H)
5,5 - 5,9 (breites s, 2H)
7,4 (s, 5H).

d) cis-2,3,3a,4,5,7a-Hexahydro[1H]indol-2-S-endo-carbonsäure

Diese Verbindung wird aus der Verbindung von Beispiel 19 c analog dem in Beispiel 1 c beschriebenen Verfahren hergestellt.

$^1$H-NMR-Daten: 1,1 - 2,9 (m, 7H)
($D_2O$) 3,9 - 4,4 (m, 2H)
5,5 - 6,4 (m, 2H)

e) N-(1-S-Carbethoxy-3-phenyl-propyl)-S-alanyl-cis-2,3,3a,4,5,7a-hexahydro/IH/indol-2-S-endo-carbonsäure

Diese Verbindung wird aus der Verbindung von Beispiel 19 d und N-(1-S-Carbethoxy-3-phenyl-propyl)-S-alanin analog dem in Beispiel 1 d beschriebenen Verfahren hergestellt.

$^1$H-NMR-Daten: 1,0 - 2,9 (m, 17H)
(nach H/D-Austausch) 3,0 - 4,6 (m, 6H)
5,2 - 6,0 (m, 2H)
7,2 (s, 5H)

**Beispiel 20**

cis-2,3,3a,4,5,7a-Hexahydro[1H]indol-2-S-endo-carbonsäure-tert.-butylester

Diese Verbindung wird aus der Verbindung von Beispiel 19 d analog dem in Beispiel 4 beschriebenen Verfahren hergestellt.

$^1$H-NMR-Daten: 1,4 (s, 9H)
1,0 - 2,7 (m, 8H)
3,2 - 4,9 (m, 2H)
5,8 (d, 2H).

**Beispiel 21**

N-(1-S-Carbethoxy-3-phenyl-propyl)-S-alanyl-cis-2,3,3a, 4,5,7a-hexahydro[1H]indol-2-S-endo-carbonsäure-tert.butylester

Diese Verbindung wird aus der Verbindung von Beispiel 20 und N-(1-S-Carbethoxy-3-phenyl-propyl)-S-alanin analog dem in Beispiel 5 beschriebenen Verfahren hergestellt.

$^1$H-NMR-Daten: 1,4 (s, 9H)
0,8 - 3,8 (m, 20H)
3,9 - 4,6 (m, 4H)
5,4 - 6,0 (m, 2H)
7,2 (s, 5H).

**Beispiel 22**

N-(1-S-Carbethoxy-3-phenyl-propyl)-S-alanyl-cis-2,3,3a, 4,5,7a-hexahydro[1H]indol-2-S-exo-carbonsäure

a) cis-2,3,3a,4,5,7a-hexahydro[1H]indol-2-S-exo-carbonsäure

Diese Verbindung wird aus der exo-Verbindung von Beispiel 19 c analog dem in Beispiel 1 c beschriebenen Verfahren hergestellt.

$^1$H-NMR-Daten: 1,0 - 2,8 (m, 1H)
($D_2O$) 3,6 - 4,4 (m, 2H)
5,5 - 6,4 (m, 2H)

b) N-(1-S-Carbethoxy-3-phenyl-propyl-S-alanyl-cis-2,3,3a, 4,5,7a-hexahydro[1H]indol-2-S-exo-carbonsäure

Diese Verbindung wird aus der Verbindung von Beispiel 22 a und N-(1-S-Carbethoxy-3-phenyl-propyl)-S-alanin analog dem in Beispiel 1 d beschriebenen Verfahren hergestellt.

$^1$H-NMR-Daten: 1, 0 - 2,9 (m, 17H)
(nach H/D-Austausch) 3,0 - 4,6 (m, 6H)
5,2 - 6,0 (m, 2H)
7,2 (s, 5H).

**Beispiel 23**

cis-2,3,3a,4,5,7a-hexahydro[1H]indol-2-S-exo-carbonsäure-tert.-butylester

Diese Verbindung wird aus der Verbindung von Beispiel 22 a analog dem in Beispiel 4 beschriebenen Verfahren hergestellt.

$^1$H-NMR-Daten: 1,4 (s, 9H)
0,9 - 2,8 (m, 8H)
3,3 - 4,9 (m, 2H)
5,4 - 5,8 (m, 2H)

**Beispiel 24**

N-(1-S-Carbethoxy-3-phenyl-propyl)-S-alanyl-cis-2,3,3a-4,5,7a-hexahydro[1]indol-2-S-exo-carbonsäure-tert.-butylester

Diese Verbindung wird aus der Verbindung von Beispiel 23 und N-(1-S-Carbethoxy-3-phenyl-propyl)-S-alanin analog dem in Beispiel 5 beschriebenen Verfahren hergestellt.

$^1$H-NMR-Daten: 1,3 (s, 9H)
0,9 - 3,7 (m, 20H)
3,8 - 4,7 (m, 4H)
5,4 - 6,0 (m, 2H)
7,2 (s, 5H).

**Beispiel 25**

N-(1-S-Carbethoxy-3-phenyl-3-oxo-propyl)-S-alanyl-cis-2,3,3a,4,5,7a-hexahydro[1H]indol-2-S-endo-carbonsäuretert.-butylester

2,5 g N-(1-S-Carbethoxy-3-phenyl-3-oxo-propyl)-S-alanin werden zusammen mit 1,2 g 1-Hydroxybenztriazol, 2,5 g cis-2,3,3a,4,5,7a-hexahydro[1]indol-2-S-endo-carbonsäure-tert.-butylester und 2 g Dicyclohexylcarbodiimid in 20 ml Dimethylformamid 1 Stunde bei 0°C und anschließend 12 Stunden bei 20 - 25°C gerührt. Die Reaktionslösung wird mit 25 ml Essigester verdünnt. Der abgeschiedene Harnstoff wird abgesaugt. Nach dem Einengen im Vakuum wird der erhaltene Rückstand in Ether aufgenommen, die etherische Lösung mit gesättigtem wäßrigen Natriumbicarbonat und mit Wasser gewaschen, getrocknet und eingedampft.

Ausbeute: 2,5 g
$^1$H-NMR-Daten: 1,2 (s, 9H)
0,9 - 2,9 (m, 15H)
3,4 - 5,0 (m, 6H)
5,2 - 6,0 (m, 2H)
7,2 - 8,2 (m, 5H)

**Beispiel 26**

N-(1-S-Carbethoxy-3-phenyl-3-oxo-propyl)-S-alanyl-cis-2,3,3a,4,5,7a-hexahydro[1H]indol-2-S-exo-carbonsäuretert.-butylester

Diese Verbindung wird aus der Verbindung von Beispiel 23 und N-(1-S-Carbethoxy-3-phenyl-3-exo-propyl)-S-alanin analog dem in Beispiel 25 beschriebenen Verfahren hergestellt.

$1_H$-NMR-Daten: 1,3 (s, 9H)
1,0 - 2,8 (m, 15H)
3,3 - 4,9 (m, 6H)
5,3 - 6,0 (m, 2H)
7,2 - 8,2 (m, 5H)

**Beispiel 27**

N-(1-S-Carbethoxy-3-phenyl-3-oxo-propyl)-S-alanyl-cis-2,3,3a,4,5,7a-hexahydro[1H]indol-2-S-endo-carbonsäuretrifluoracetat

1,3 g der Verbindung aus Beispiel 25 werden in 5 ml Trifluoressigsäure 2 Stunden bei 20 - 25°C gerührt. Man

engt die Lösung im Vakuum ein, verreibt den Rückstand mit Diisopropylether und saugt ab

Ausbeute: 0,4 g

$^1$H-NMR-Daten: 1,0 - 3,6 (m, 15H)
(nach H/D-Austausch) 3,9 - 4,6 (m, 6H)
5,2 - 5,9 (m, 2H)
7,3 - 8,1 (m, 5H).

**Beispiel 28**

N-(1-S-Carbethoxy-3-phenyl-3-oxo-propyl)-S-alanyl-cis-2,3,3a,4,5,7a-hexahydro[1H]indol-2-S-exo-carbonsäurehydrochlorid

0,5 g der Verbindung aus Beispiel 26 werden in 5 ml Methylenchlorid gelöst, mit Chlorwasserstoffgas gesättigt und 16 Stunden bei 20 - 25°C stehengelassen. Man engt im Vakuum ein und verreibt den Rückstand mit Diisopropylether und saugt ab.

Ausbeute: 0,3 g.

$^1$H-NMR-Daten: 0 9 - 3 5 (m 15H)
(nach H/D-Austausch) 3 9 - 4 8 (m, 6H)
5,3 - 6,0 (m, 2H)
7,1 - 8,0 (m, 5H)

**Beispiel 29**

N-(1-S-Carboxy-3-phenyl-3-oxo-propyl)-S-alanyl-cis-2,3,3a,4,5,7a-hexahydro/IH/indol-2-S-endo-carbonsäure

0,5 g der Verbindung aus Beispiel 27 werden analog der im Beispiel 10 beschriebenen Verfahrensweise mit 2 äquivalenten Kaliumhydroxid umgesetzt Ausbeute: 0,25 g.

$^1$H-NMR-Daten: 1,1 - 3,6 (m, 12H)
(nach H/D-Austausch) 3,7 - 4,6 (m, 4H)
5,2 - 5,9 (m, 2H)
7,1 - 8,0 (m, 5H).

**Beispiel 30**

N-(1-S-Carboxy-3-phenyl-3-oxo-propyl)-S-alanyl-cis-2,3,3a,4,5,7a-hexahydro[1H]indol-2-S-exo-carbonsäure

0,5 g der Verbindung aus Beispiel 28 werden analog der in Beispiel 10 beschriebenen Verfahrensweise mit 2 Äquivalenten Kaliumhydroxid umgesetzt.

Ausbeute: 0,3 g

$^1$H-NMR-Daten: 1,0 - 3,5 (m, 12H)
(nach H/D-Austausch) 3,6 - 4,8 (m, 4H)
5,2 - 5,9 (m, 2H)
7,2 - 8,0 (m, 5H)

**Beispiel 31**

S-Alanyl-cis-2,3,3a,4,5,7a-hexahydro[1H]indol-2-S-endo-carbonsäure-tert.-butylester

a) N-Methylsulfonylethyloxycarbonyl (MSC)-S-alanyl-cis-2,3,3a,4,5,7a-hexahydro[1]indol-2-S-endo-carbonsäuretert.-butylester

Zu einer Lösung von 10 g MSC-Ala-OH in 50 ml Dimethylformamid werden 6,7 g 1-Hydroxy-benztriazol und 14,7 g cis-2,3,3a,4,5,7a-hexahydro[1H]indol-2-S-endo-carbon-säure-tert.-butylester gegeben. Der pH-Wert wird mit N-Ethylmorpholin auf 8,0 eingestellt. Das Gemisch wird im Eisbad gekühlt und mit 10,5 g Dicyclohexylcarbodiimid versetzt. Man rührt 15 Stunden bei 20-25°C. Der ausgefallene Harnstoff wird abgesaugt, das Filtrat im Vakuum eingeengt und in Essigester aufgenommen. Die organische Phase wird mit nacheinander Kaliumhydrogensulfat-, Kaliumhydrogencarbonat- und Natriumchloridlösung gewaschen, getrocknet und eingedampft. Der Rückstand wird an Kieselgel mit Essigester /Cyclohexan 1:1 chromatographiert.

Ausbeute: 10 g

$^1$H-NMR-Daten: 1,4 (s, 9H)
1,3 (d, 3H)

1,1 - 2,6 (m, 7H)
3,0 (s, 3H)
3,2 - 3,5 (m, 2H)
3,5 - 4,9 (m, 5H)
5,6 - 5,9 (m, 2H)

b) S-Alanyl-cis-2,3,3a,4,5,7a-hexahydro[1]indol-2-S-endo-carbonsäure-tert.-butylester

2,0 g der Verbindung aus Beispiel 31 a werden in 15 ml Methanol und 1,5 ml Wasser gelöst. Es wird mit 2n Natronlauge auf pH 13 gebracht und 2 Stunden bei Raumtemperatur gerührt. Danach wird mit 2n Salzsäure neutralisiert, das Methanol im Vakuum abgedampft die wäßrige Phase mit Essigester extrahiert, die Essigesterlösung mit Wasser gewaschen, getrocknet und eingeengt. Der Rückstand wird über Kieselgel mit Essigester als Elutionsmittel filtriert.

Ausbeute: 0,8 g

$^1$H-NMR-Daten: 1,4 (s, 9H)
1,3 (d, 3H)
1,0 - 2,4 (m, 7H)
3,5 - 4,8 (m, 3H)
5,5 - 5,9 (m, 2H)

**Beispiel 32**

S-Alanyl-cis-2,3,3a,4,5,7a-hexahydro[1H]indol-2-S-exo-carbonssäure-tert.-butylester

Diese Verbindung wird aus der Verbindung von Beispiel 23 analog dem in Beispiel 31 beschriebenen Verfahren hergestellt.

$^1$H-NMR-Daten: 1,2 (d, 3H)
1,4 (s, 9H)
0,9 - 2,3 (m, 7H)
3,4 - 4,7 (m, 3H)
5,6 - 5,9 (m, 2H).

**Beispiel 33**

N-(1-S-Carbethoxy-3-oxo-3-phenyl-propyl)-S-alanyl-cis-2,3,3a,4,5,7a-hexahydro[1H]indol-2-S-endo-carbonsäuretert.-butylester

5 m Mol der Verbindung aus Beispiel 31b werden zusammen mit 5 m Mol 3-Benzoyl-acrylsäureethylester und 5 Tropfen Triethylamin in 50 ml wasserfreiem Ethanol gelöst und das Gemisch 24 Stunden bei 20 bis 25°C gerührt. Es wird zur Trockene eingedampft und der Rückstand in Essigester aufgenommen. Nun wird mit Wasser gewaschen, getrocknet und eingedampft.

Das Diastereomerengemisch wird an Kieselgel mit Essigester/Cyclohexan als Elutionsmittel chromatographiert. Die $1_H$-NMR-Daten stimmen mit den Daten der Verbindung aus Beispiel 25 überein.

**Beispiel 34**

N-(1-S-Carbethoxy-3-oxo-3-phenyl-propyl)-S-alanyl-cis-2,3,3a,4,5,7a-hexahydro/IH/indol-2-S-exo-carbonsäure-tert.butylester

Diese Verbindung wird aus der Verbindung von Beispiel 32 mit Benzoyl-acrylsäureethylester analog dem in Beispiel 33 beschriebenen Verfahren hergestellt.

**Beispiel 35**

N-(1-S-Carbethoxy-3-phenyl-propyl)-S-alanyl-cis-2,3,3a-4,5,7a-hexahydro/IH/indol-2-S-endo-carbonsäure-tert.butylester

5 m Mol S-Alanyl-cis-2,3,3a,4,5,7a-hexahydro[1H]indol-2-S-endo-carbonsäure-tert.-butylester werden in 15 ml wasserfreiem Ethanol gelöst. Man stellt die Lösung mit ethanolischem Kaliumhydroxid auf pH 7,0 und gibt 0,7 g pulverisiertes Molekularsieb (4Å) und anschließend 5 m Mol 2-Keto-4-phenyl-buttersäureethylester dazu. Es wird eine Lösung von 0,6 g Natriumcyanborhydrid in 6 ml wasserfreiem Ethanol langsam zugetropft. Nach einer Reaktionszeit von 20 Stunden bei 20 bis 25°C wird die Lösung filtriert und das Lösungsmittel abdestilliert. Der Rückstand wird in Essigester/Wasser aufgenommen. Nach dem Eindampfen der Essigesterphasen wird

der Rückstand an Kieselgel mit Essigester/Cyclohexan 1:4 chromatographiert

Die $^1$H-NMR-Daten stimmen mit den Daten der Verbindung aus Beispiel 21 überein.

**Beispiel 36**

N-(1-S-Carbethoxy-3-phenyl-propyl)-S-alanyl-cis-2,3,3a,4, 5,7a-hexahydro[1H]indol-2-S-exo-carbonsäure-tert.-butylester

Diese Verbindung wird aus der Verbindung von Beispiel 32 und 2-Keto-4-phenyl-buttersäureethylester analog dem in Beispiel 35 beschriebenen Verfahren hergestellt.

Die $^1$H-NMR-Daten stimmen mit den Daten der Verbindung aus Beispiel 24 überein.

**Beispiel 37**

N-(1-S-Carbethoxy-3-oxo-3-phenyl-propyl)-S-alanyl-cis-2,3,3a,4,5,7a-hexahydro/IH/indol-2-S-endo-carbonsäuretert.-butylester

10 mMol Acetophenon, 10 mMol Glyoxylsäureethylester und 10 mMol S-Alanyl-cis-2,3,3a,4,5,7a-hexahydro[1H]indol -2-S-endo-carbonsäure-tert.-butylester werden in 30 ml Eisessig 36 Stunden auf 45°C erhitzt. Nach dem Einengen im Vakuum wird mit Natriumbicarbonatlösung neutral gestellt und mit Essigester extrahiert. Die Essigesterphase wird eingeengt und an Kieselgel mit Essigester /Cyclohexan 1:1 als Elutionsmittel chromatographiert.

Die $^1$H-NMR-Daten stimmen mit den Daten der Verbindung aus Beispiel 25 überein.

**Beispiel 38**

N-(1-S-Carbethoxy-3-oxo-3-phenyl-propyl)-S-alanyl-cis-2,3,3a,4,5,7a-hexahydro[1H]indol-2-S-exo-carbonsäuretert.-butylester

Diese Verbindung wird aus der Verbindung von Beispiel 32 und Glyoxylsäureethylester und Acetophenon analog den im Beispiel 37 beschriebenen Verfahren hergestellt.

Die $^1$H-NMR-Daten stimmen mit den Daten der Verbindung aus Beispiel 24 überein.

**Beispiel 39**

N-(1-S-Carbethoxy-3-R,S-hydroxy-3-phenyl-propyl-)-S-alanyl-cis-2,3,3a,4,5,7a-hexahydro[1]indol-2-S-endo-carbonsäure

0,5 g N-(1-S-Carbethoxy-3-oxo-3-phenyl-propyl)-S-alanyl-cis-2,3,3a,4,5,7a-hexahydro[1]indol-2-S-endo-carbonsäure werden in 5 ml wäßrigem Ethanol gelöst und 0,1 g Natriumborhydrid zugegeben. Es wird 14 Stunden bei Raumtemperatur gerührt. Danach wird mit Essigester versetzt, die Essigesterlösung mit Wasser gewaschen, getrocknet und eingedampft. Das Rohprodukt wird über Kieselgel mit Essigester/Methanol 9:1 als Elutionsmittel filtriert.

Ausbeute: 0,3 g.

$^1$H-NMR-Daten: 1,0 - 3,5 (m, 15H)
(nach H/D-Austausch) 3,8 - 4,8 (m, 7H)
5,3 - 5,8 (m, 2H)
7,2 (s, 5H).

**Beispiel 40**

N-(1-S-Carbethoxy-3-R,S-hydroxy-3-phenyl-propyl)-S-alanyl-cis-2,3,3a,4,5,7a-hexahydro[1]indol-2-S-exo-carbonsäure

0,5 g N-(1-S-Carbethoxy-3-oxo-phenyl-propyl)-S-alanyl-cis-2,3,3a,4,5,7a-hexahydro[1]indol-2-S-exo-carbonsäure werden mit 0,1 g Natriumborhydrid analog dem in Beispiel 39 beschriebenen Verfahren umgesetzt.

Ausbeute: 0,2 g

$^1$H-NMR-Daten: 0,9 - 3,4 (m, 15H)
(nach H/D-Austausch) 3,7 - 4,9 (m, 7H)
5,2 - 5,9 (m, 2H)
7,2 (s, 5H).

**Patentansprüche**

für die Vertragsstaaten BE, CH, DE, FR, GB, IT, LI, LU, NL, SE.

1. Verbindungen der Formel I,

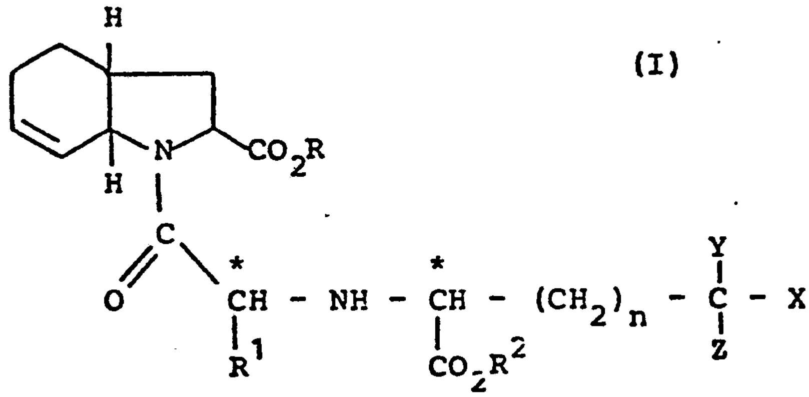

(I)

in welcher
n = 0 oder 1,
R = Wasserstoff, ($C_1$ bis $C_6$)-Alkyl oder Aralkyl mit 7 bis 9 C-Atomen,
$R^1$ = Wasserstoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{10}$)-Aryl oder teilhydriertes ($C_6$ bis $C_{10}$)-Aryl, das jeweils durch ($C_1$ bis $C_4$)-Alkyl, ($C_1$ oder $C_2$)-Alkoxy oder Halogen substituiert sein kann, ($C_6$ bis $C_{10}$) Aryl-($C_1$ bis $C_4$)-alkyl oder ($C_7$ bis $C_{11}$) Aroyl-$C_1$-alkyl, die beide wie vorstehend definiert im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,
$R^2$ = Wasserstoff, ($C_1$ bis $C_6$)-Alkyl, ($C_2$ bis $C_6$)-Alkenyl oder Aryl-($C_1$ bis $C_4$)-alkyl,
Y = Wasserstoff oder Hydroxy,
Z = Wasserstoff oder
Y und Z = zusammen Sauerstoff und
X = ($C_1$ bis $C_6$)-Alyl, ($C_2$ bis $C_6$)-Alkenyl, ($C_5$ bis $C_9$)-Cyclcalkyl, ($C_6$ bis $C_{10}$)-Aryl, das durch ($C_1$ bis $C_4$), Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino oder Methylendioxy mono-, di- oder trisubstituiert sein kann oder 3-Indolyl
sind,
sowie deren physiologisch unbedenkliche Salze.

2. Verbindungen der Formel I gemäß Anspruch 1, dadurch gekennzeichnet, daß die Wasserstoffatome an den Brückenkopf-C-Atomen 3 a und 7 a zueinander cis-konfiguriert sind.

3. Verbindungen der Formel I gemäß Ansprüche 1 oder 2, dadurch gekennzeichnet, daß das C-Atom in Position 2 des Hexahydroindolsystems sowie die mit einem Stern markierten C-Atome der Seitenkette jeweils S-Konfiguration aufweisen.

4. Verbindungen der Formel I gemäß einem der Ansprüche 1 bis 3, dadurch gekennzeichnet, daß
n = 1
R = Wasserstoff oder ($C_1$ bis $C_4$)-Alkyl,
$R^1$ = Wasserstoff, ($C_1$ bis $C_3$)-Alkyl, ($C_2$ oder $C_3$)-Al-kenyl, Benzyl, Phenethyl, 4-Amino-butyl oder Benzoylmethyl
$R^2$ = Wasserstoff, ($C_1$ bis $C_4$)-Alkyl oder Benzyl,
X = Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, Di-($C_1$ bis $C_4$)alkyl-amino, Nitro oder Methylendioxy mono- oder disubstituiert oder, im Falle von Methoxy, trisubstituiert sein kann, bedeuten.

5. Verbindungen der Formel I gemäß einem der Ansprüche 1 bis 4, dadurch gekennzeichnet, daß R = Wasserstoff, $R^1$ = Methyl und X = Phenyl bedeuten.

6. Verbindungen der Formel I gemäß einem der Ansprüche 1 bis 5, dadurch gekennzeichnet, daß R = Wasserstoff, $R^1$ = Methyl, X = Phenyl und $R^2$ = Wasserstoff oder Ethyl bedeuten.

7. Verbindung der Formel I gemäß Anspruch 2, dadurch gekennzeichnet, daß die Carboxygruppe am C-Atom in Position 2 des Hexahydroindolsystems endoständig zum Bicyclus orientiert ist und

das vorstehend genannte C-Atom sowie die mit einem Stern markierten C-Atome der Seitenkette jeweils S-Konfiguration aufweisen,
n = 1,
R = Wasserstoff,
$R^1$ = Methyl,
$R^2$ = Ethyl,
Y und Z jeweils = Wasserstoff und
X = Phenyl bedeuten.

8. Verfahren zur Herstellung der Verbindungen der Formel I gemäß den Ansprüchen 1 bis 7, dadurch gekennzeichnet,

a) daß man eine Verbindung der Formel II,

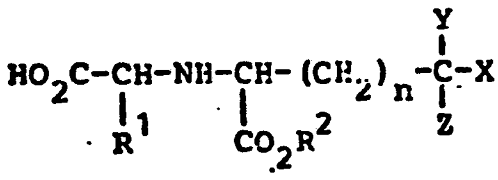

(II)

worin n, $R^1$, $R^2$, X, Y und Z die Bedeutung wie in Formel I haben, mit einer Verbindung der Formel III,

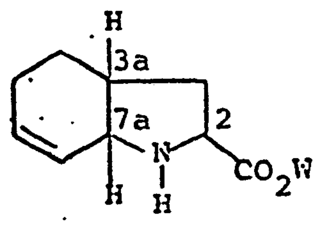

(III)

in welcher
W = Wasserstoff oder einen sauer abspaltbaren Rest bedeutet, umsetzt und anschließend gegebenenfalls W und/oder $R^2$ unter Bildung der freien Carboxygruppen abspaltet, oder

b) daß man zur Herstellung der Verbindungen der Formel I, in der Y und Z zusammen Sauerstoff bedeuten,

$b_1$) eine Verbindung der Formel IV,

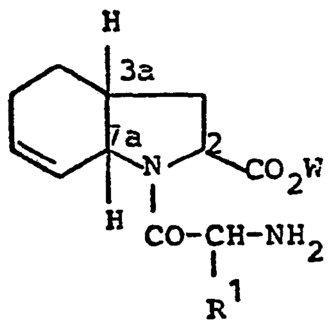

(IV)

in welcher $R^1$ die Bedeutung wie in Formel I und W die Bedeutung wie in Formel III besitzen, mit einer Verbindung der Formel V,

$R^2O_2C-CH=CH-CO-X$ V

worin $R^2$ und X die Bedeutungen wie in Formel I haben, umsetzt und anschließend gegebenenfalls W und/oder $R^2$ unter Bildung der freien Carboxygruppen abspaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$OHC-CO_2R^2$ $X-CO-CH_3$
(VI) (VII)

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

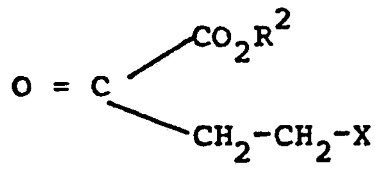

(VIII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet, oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert,

und die nach a) bis d) erhaltenen Verbindungen der Formel I, in welcher R für Wasserstoff steht, gegebenenfalls in Ester der Formel I, worin R ($C_1$bis $C_6$)-Alkyl oder ($C_7$ bis $C_9$)-Aralkyl bedeutet, überführt, und Verbindungen der Formel I gegebenenfalls in ihre physiologisch unbedenklichen Salze überführt.

9. Verbindung gemäß einem der Ansprüche 1 bis 7 zur Anwendung als Heilmittel.

10. Heilmittel, enthaltend eine Verbindung gemäß einem der Ansprüche 1 bis 7.

11. Verbindungen der Formel III,

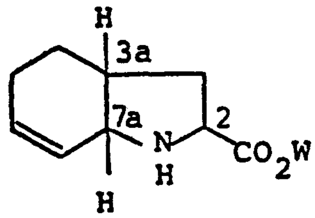

(III)

in der die H-Atome an den C-Atomen 3 a und 7 a zueinander cis-konfiguriert sind und die Gruppe $-CO_2W$ an C-Atom 2 exo- oder endo-ständig zum bicyclischen Ringsystem orientiert ist und worin

W = Wasserstoff oder einen sauer abspaltbaren Rest bedeutet.

12. Verfahren zur Herstellung von Verbindungen der Formel III gemäß Anspruch 11, dadurch gekennzeichnet, daß man

Verbindungen der Formel IX,

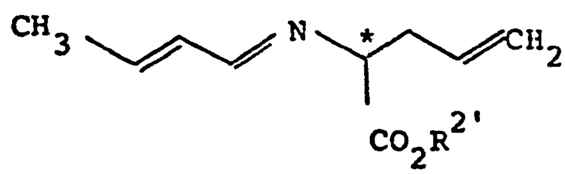

(IX)

in welcher $R^{2'}$ ($C_1$ bis $C_8$)-Alkyl oder ($C_7$ oder $C_8$)-Aralkyl bedeutet, mit Acylierungsmitteln, die die Gruppe $-CO-R^3$, worin der Rest $R^3$ für ($C_1$ bis $C_8$)-Alkyl, ($C_5$ bis $C_8$)-Cycloalkyl, ($C_2$ bis $C_6$)-Alkenyl, ($C_1$ bis $C_6$)-Alkoxy, Aryl, Aryloxy, Aryl-($C_1$ bis $C_4$)-alkyl oder Aryl-($C_1$ bis $C_4$)-alkoxy steht, übertragen, zu Verbindungen der Formel X,

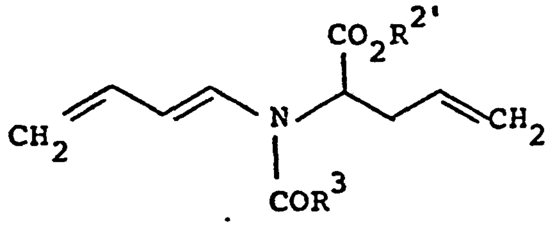

(X)

in welcher $R^{2'}$ und $R^3$ vorstehende Bedeutung haben, umsetzt,
diese zu einem Gemisch stereoisomerer Verbindungen der Formel XI,

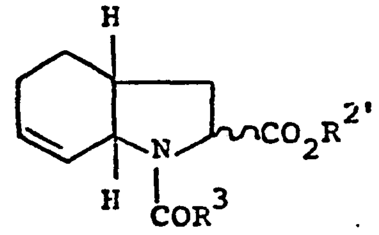

(XI)

in welcher $R^{2'}$ und $R^3$ vorstehende Bedeutung haben und die Wasserstoffatome an den Brückenkopf-C-Atomen cis-konfiguriert sind, cyclisiert,

dieses Stereoisomerengemisch, in dem je nachdem, ob von R-, S-konfigurierten oder racemischen Verbindungen der Formel IX ausgegangen wurde, Diastereomerenpaare der Formeln XI b und d bzw. XI a und c, bzw. Gemische der Formeln IX a - d,

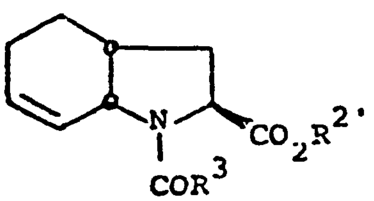

(XI c)

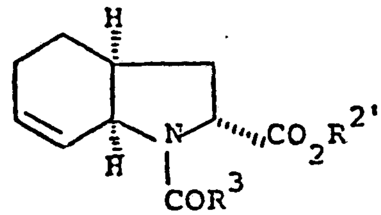

(XI d)

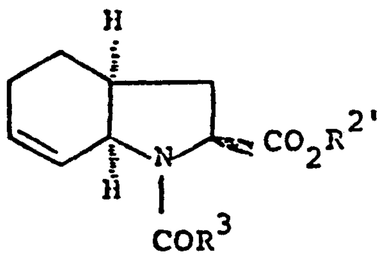

(XI a)

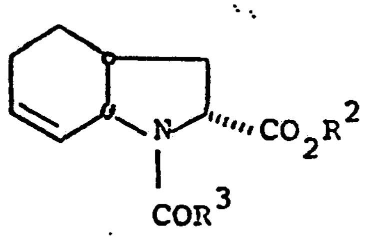

(XI b)

in welchen $R^{2'}$ und $R^3$ jeweils die vorstehende Bedeutung haben vorliegen, gegebenenfalls nach vorheriger Auftrennung in Enantiomere, Diastereomerenpaare oder Racemate, zu Verbindungen der Formel III gemäß Anspruch 11, in welcher W für Wasserstoff steht, verseift und

diese gegebenenfalls zu Verbindungen der Formel III, in welcher W für einen sauer abspaltbaren Rest steht, verestert.

13. Verwendung einer Verbindung gemäß Anspruch 11 zur Herstellung einer Verbindung gemäß einem der Ansprüche 1 bis 7.

14. Verbindung gemäß Anspruch 9 zur Anwendung in Kombination mit einem Diuretikum.

15. Heilmittel, enthaltend eine Verbindung gemäß einem der Ansprüche 1 bis 7 in Kombination mit einem Diuretikum.

16. Verbindung gemäß Anspruch 9 oder 14 zur Anwendung als Heilmittel bei der Behandlung des Bluthochdrucks.

**Patentansprüche**

für den Vertragsstaat AT

1. Verfahren zur Herstellung von Verbindungen der Formel I

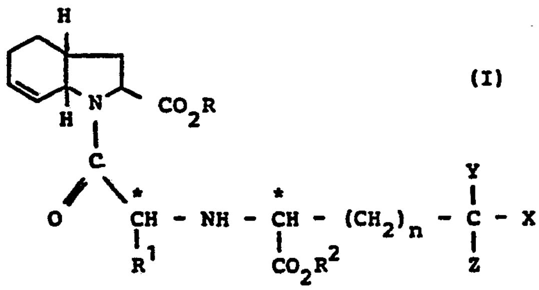

(I)

in welcher

n = 0 oder 1,

R = Wasserstoff, $C_1$ bis $C_6$)-Alkyl oder Aralkyl mit 7 bis 9 C-Atomen,

$R^1$ = Wasserstoff oder ($C_1$ bis $C_6$)-Alkyl, das gegebenenfalls durch Amino, ($C_1$ bis $C_4$)-Acylamino oder Benzoylamino substituiert sein kann, ($C_2$ bis $C_6$)-Alkenyl, ($C_5$ bis $C_9$)-Cycloalkyl, ($C_5$ bis $C_9$)-Cycloalkenyl, ($C_5$ bis $C_7$)-Cycloalkyl-($C_1$ bis $C_4$)-alkyl, ($C_6$ bis $C_{10}$)-Aryl oder teilhydriertes ($C_6$ bis $C_{10}$)-Aryl, das jeweils durch ($C_1$ bis $C_4$)-Alkyl, ($C_1$ oder $C_2$)-Alkoxy oder Halogen substituiert sein kann, ($C_6$ bis $C_{10}$)-Aryl ($C_1$ bis $C_4$)-alkyl oder ($C_7$ bis $C_{11}$)-Aroyl-$C_1$-alkyl die beide wie vorstehend definiert, im Arylrest substituiert sein können, ein mono- bzw. bicyclischer Heterocyclen-Rest mit 5 bis 7 bzw. 8 bis 10 Ringatomen, wovon 1 bis 2 Ringatome Schwefel- oder Sauerstoffatome und/oder wovon 1 bis 4 Ringatome Stickstoffatome darstellen, oder eine Seitenkette einer natürlich vorkommenden Aminosäure,

$R^2$ = Wasserstoff, ($C_1$ bis $C_6$)-Alkyl, ($C_2$ bis $C_6$)-Alkenyl oder Aryl-($C_1$ bis $C_4$)-alkyl,

Y = Wasserstoff oder Hydroxy,

Z = Wasserstoff oder

Y und Z = zusammen Sauerstoff und

X = ($C_1$ bis $C_6$)-Alkyl, ($C_2$ bis $C_6$)-Alkenyl, ($C_5$ bis $C_6$)-Cycloalkyl, ($C_6$ bis $C_{10}$)-Aryl, das durch ($C_1$ bis $C_4$)-Alkyl, ($C_1$ bis $C_4$)-Alkoxy, Hydroxy, Halogen, Nitro, Amino, ($C_1$ bis $C_4$)-Alkylamino, Di-($C_1$ bis $C_4$)alkyl-amino oder Methylendioxy mono-, di- oder trisubstituiert sein kann, oder 3-Indolyl

bedeuten,

sowie deren physiologisch unbedenkliche Salze, dadurch gekennzeichnet,

a) daß man eine Verbindung der Formel II,

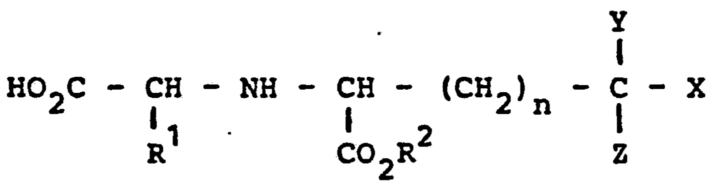

(II)

worin n, $R^1$, $R^2$, X, Y und Z die Bedeutung wie in Formel I haben mit einer Verbindung der Formel III,

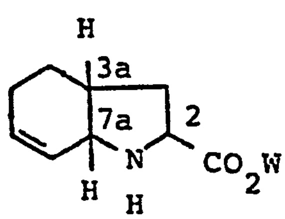

(III)

in welcher

W = Wasserstoff oder einen sauer abspaltbaren Rest bedeutet, umsetzt und anschließend gegebenenfalls W und/oder $R^2$ unter Bildung der freien Carboxygruppen abspaltet, oder,

b) daß man zur Herstellung der Verbindungen der Formel I, in der Y und Z zusammen Sauerstoff bedeuten,

$b_1$) eine Verbindung der Formel IV,

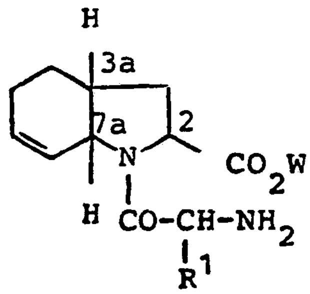

(IV)

in welcher $R^1$ die Bedeutung wie in Formel I und W die Bedeutung wie in Formel III besitzen, mit einer Verbindung der Formel V,

$R^2O_2C\text{-}CH{=}CH\text{-}CO\text{-}X$ V

worin $R^2$ und X die Bedeutungen wie in Formel I haben, umsetzt und anschließend gegebenenfalls W und/oder $R^2$ unter Bildung der freien Carboxygruppen abspaltet, oder

$b_2$) eine Verbindung der unter $b_1$) genannten Formel IV mit einer Verbindung der allgemeinen Formel VI, worin $R^2$ die Bedeutung wie in Formel I hat, und mit einer Verbindung der allgemeinen Formel VII,

$OHC\text{-}CO_2R^2$ C-CO-$CH_3$

(VI) (VII)

worin X die Bedeutung wie in Formel I hat, umsetzt und anschließend gegebenenfalls W und/oder $R^2$ unter Bildung der freien Carboxygruppen abspaltet, oder

c) daß man zur Herstellung von Verbindungen der Formel I, in der Y und Z jeweils Wasserstoff bedeuten, eine Verbindung der unter $b_1$) genannten Formel IV mit einer Verbindung der Formel VIII,

$OHC\text{-}CO_2R^2$ C-CO-$CH_3$

(VI) (VII)

worin $R^2$ und X die Bedeutungen wie in Formel I besitzen, umsetzt, die erhaltenen Schiff-Basen reduziert und anschließend gegebenenfalls W und/oder $R^2$ unter Bildung der freien Carboxygruppen abspaltet oder

d) daß man zur Herstellung von Verbindungen der Formel I, in der Y = Hydroxy und Z = Wasserstoff bedeuten, eine Verbindung der Formel I, in der Y und Z zusammen Sauerstoff bedeuten, mit einem komplexen Boranat oder Boran-Amin-Komplex reduziert.

und die nach a) bis d) erhaltenen Verbindungen der Formel I, in welcher R für Wasserstoff steht, gegebenenfalls in Ester der Formel I, worin R ($C_1$ bis $C_6$)-Alkyl oder ($C_7$ bis $C_9$)-Aralkyl bedeutet, überführt und Verbindungen der Formel I gegebenenfalls in ihre physiologisch unbedenklichen Salze überführt.

2. Verfahren gemäß Anspruch 1, dadurch gekennzeichnet, daß Verbindungen der Formel I hergestellt werden, in denen die Wasserstoffatome an den Brückenkopf-C-Atomen 3a und 7a zueinander cis-konfiguriert sind.

3. Verfahren gemäß einem der Ansprüche 1 oder 2, dadurch gekennzeichnet, daß Verbindungen der Formel I hergestellt werden, in denen das C-Atom in Position 2 des Hexahydroindolsystems sowie die mit einem Stern markierten C-Atome der Seitenkette jeweils S-Konfiguration aufweisen.

4. Verfahren gemäß den Ansprüchen 1 bis 3, dadurch gekennzeichnet, daß in den Verbindungen der Formel I
n = 1
R = Wasserstoff oder ($C_1$ bis $C_4$)-Alkyl,
$R^1$ = Wasserstoff, ($C_1$ bis $C_3$)-Alkyl, ($C_2$ oder $C_3$)-Al-kenyl, Benzyl, Phenethyl, 4-Amino-butyl oder Benzoylmethyl
$R^2$ = Wasserstoff, ($C_1$ bis $C_4$)-Alkyl oder Benzyl,
X = Phenyl, das durch ($C_1$ oder $C_2$)-Alkyl, ($C_1$ oder $C_2$)-Alkoxy, Hydroxy, Fluor, Chlor, Brom, Amino, ($C_1$ bis $C_4$)-Alkylamino, D-($C_1$ bis $C_4$)alkyl-amino, Nitro oder Methylendioxy mono- oder disubstituiert oder, im Falle von Methoxy, trisubstituiert sein kann,
bedeuten.

5. Verfahren gemäß einem der Ansprüche 1 bis 4, dadurch gekennzeichnet, daß in den Verbindungen der Formel I R = Wasserstoff, $R^1$ = Methyl und X = Phenyl bedeuten.

6. Verfahren gemäß einem der Ansprüche 1 bis 5, dadurch gekennzeichnet, daß in den Verbindungen der Formel I R = Wasserstoff, $R^1$ = Methyl, X = Phenyl und $R^2$ = Wasserstoff oder Ethyl bedeuten.

7. Verfahren gemäß Anspruch 2, dadurch gekennzeichnet, daß eine Verbindung der Formel I hergestellt wird, in der die Carboxygruppe am C-Atom in Position 2 des Hexahydroindolsystems endoständig zum Bicyclus orientiert ist und das vorstehend genannte C-Atom sowie die mit einem Stern markierten C-Atome der Seitenkette jeweils S-Konfiguration aufweisen,
n = 1,
R = Wasserstoff,

$R^1$ = Methyl,
$R^2$ = Ethyl,
Y und Z jeweils = Wasserstoff und
X = Phenyl bedeuten.

8. Verfahren zur Herstellung einer Verbindung der Formel I gemäß einem der Ansprüche 1 bis 7 zur Verwendung als Heilmittel.

9. Verfahren zur Herstellung eines Mittels, enthaltend eine Verbindung der Formel I, dadurch gekennzeichnet, daß man die Verbindung der Formel I in eine geeignete Zubereitungsform bringt.

10. Verfahren zur Herstellung von Verbindungen der Formel III

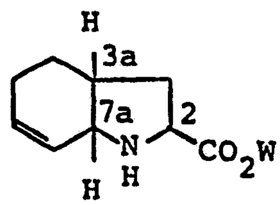

(III)

in der die H-Atome an den C-Atomen 3a und 7a zueinander cis-konfiguriert sind und die Gruppe $-CO_2W$ an C-Atom 2-exo- oder endo-ständig zum bicyclischen Ringsystem orientiert ist und worin W = Wasserstoff oder einen sauer abspaltbaren Rest bedeutet, dadurch gekennzeichnet, daß man Verbindungen der Formel IX,

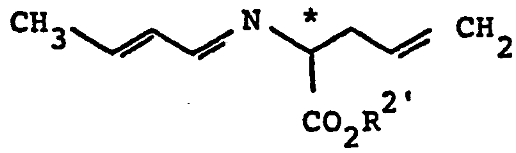

(IX)

in welcher $R^2$ ($C_1$ bis $C_8$)-Alkyl oder ($C_7$ oder $C_8$)-Ar-alkyl bedeutet, mit Acylierungsmitteln, die die Gruppe $-CO-R^3$, worin der Rest $R^3$ für ($C_1$ bis $C_6$)-Alkyl, ($C_5$ bis $C_9$)-Cycloalkyl, ($C_2$ bis $C_6$)-Alkenyl, ($C_1$ bis $C_6$)-Alkoxy, Aryl, Aryloxy, Aryl-($C_1$ bis $C_4$)-alkyl oder Aryl-($C_1$ bis $C_4$)-alkoxy steht, übertragen, zu Verbindungen der Formel X,

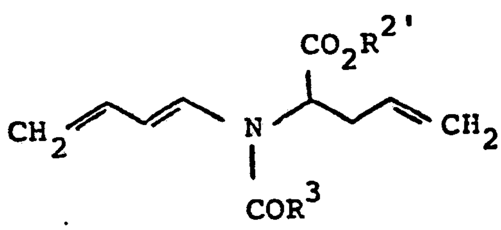

(X)

in welcher $R^{2'}$ und $R^3$ vorstehende Bedeutung haben, umsetzt,
diese zu einem Gemisch stereoisomerer Verbindungen der Formel XI,

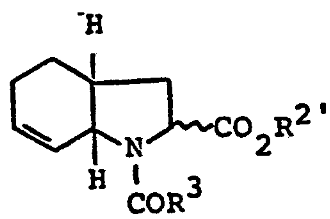

(XI)

in welcher $R^{2'}$ und $R^3$ vorstehende Bedeutung haben und die Wasserstoffatome an den Brückenkopf-C-Atomen ciskonfiguriert sind, cyclisiert, dieses Stereoisomerengemisch, in dem je nachdem, ob von R-, S-konfigurierten oder racemischen Verbindungen der Formel IX ausgegangen wurde, Diastereomerenpaare der Formeln XIb und d bzw. XIa und c, bzw. Gemische der Formeln IXa - d,

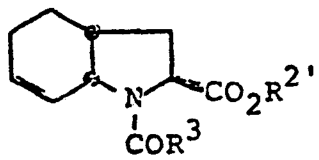

(XI c)

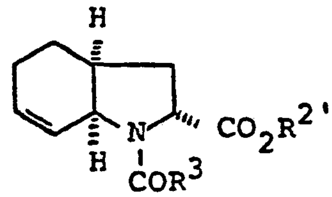

(XI d)

in welchen $R^{2'}$ und $R^3$ jeweils die vorstehende Bedeutung haben vorliegen, gegebenenfalls nach vorheriger Auftrennung in Enantiomere, Diastereomerenpaare oder Racemate, zu Verbindungen der Formel III gemäß Anspruch 11, in welcher W für Wasserstoff steht, verseift und

diese gegebenenfalls zu Verbindungen der Formel III, in welcher W für einen sauer abspaltbaren Rest steht, verestert.

11. Verfahren zur Herstellung einer Verbindung der Formel I gemäß einem der Ansprüche 1 bis 7, zur Verwendung in Kombination mit einem Diuretikum als Heilmittel.

12. Verfahren zur Herstellung eines Mittels, enthaltend eine Verbindung der Formel I in Kombination mit einem Diuretikum, dadurch gekennzeichnet, daß man die Verbindung der Formel I zusammen mit einem Diuretikum in eine geeignete Zubereitungsform bringt.

**Claims**

for the contracting States: BE, CH, DE, FR, GB, IT, LI, LU, NL, SE

1. Compounds of the formula I,

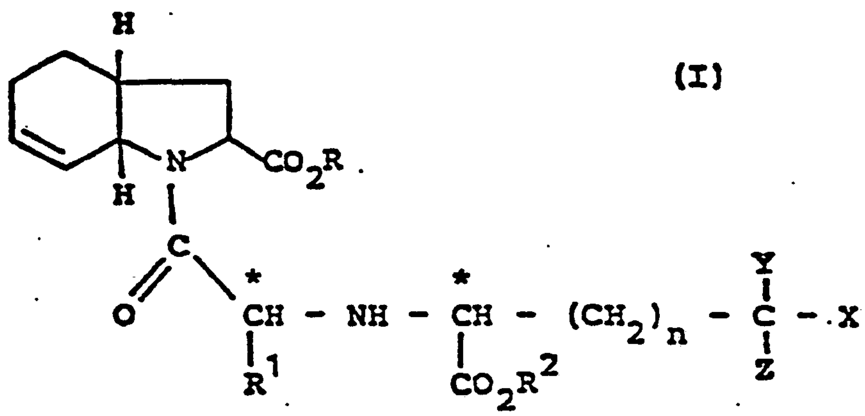

(I)

in which

n denotes 0 or 1,

R denotes hydrogen, ($C_1$ to $C_6$)-alkyl or aralkyl having 7 to 9 C atoms,

$R^1$ denotes hydrogen or ($C_1$ to $C_6$)-alkyl, which can optionally be substituted by amino, ($C_1$ to $C_4$)-acyl-amino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to $C_4$)-alkyl, ($C_6$ to $C_{10}$)-aryl or partially hydrogenated ($C_6$ to $C_{10}$)-aryl, which each can be substituted by ($C_1$ to $C_4$)-alkyl, ($C_1$ or $C_2$)-alkoxy or halogen, ($C_6$ to $C_{10}$)-aryl-($C_1$ to $C_4$)-alkyl or ($C_7$ to $C_{11}$)-aroyl-$C_1$-alkyl, both of which can be substituted in the aryl radical as defined in the aforegoing, a monocyclic or bicyclic heterocyclic radical having 5 to 7 or 8 to 10 ring atoms respectively, of which 1 to 2 ring atoms represent sulfur or oxygen atoms and/or of which 1 to 4 ring atoms represent nitrogen atoms, or a side chain of a naturally occurring aminoacid,

$R^2$ denotes hydrogen, ($C_1$ to $C_6$)-alkyl, ($C_2$ to $C_6$)-alkenyl or aryl-($C_1$ to $C_4$)-alkyl,

Y denotes hydrogen or hydroxyl,

Z denotes hydrogen or

Y and Z together denote oxygen and

X denotes ($C_1$ to $C_6$)-alkyl, ($C_2$ to $C_6$)-alkenyl, ($C_5$ to $C_9$)-cycloalkyl, ($C_6$ to $C_{10}$)-aryl, which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino or methylenedioxy, or 3-indolyl, and

its physiologically acceptable salts.

2. Compounds of the formula I as claimed in claim 1, characterized in that the hydrogen atoms on the bridgehead C-atoms 3a and 7a have the cis configuration relative to one another.

3. Compounds of the formula I as claimed in either of claims 1 or 2, characterized in that the C atom in position 2 of the hexahydroindole system and the C atom in the side chain marked with an asterisk each have the S configuration.

4. Compounds of the formula I as claimed in any of claims 1 to 3,

n denotes 1,

R denotes hydrogen or ($C_1$ to $C_4$)-alkyl,

$R^1$ denotes hydrogen, ($C_1$ to $C_3$)-alkyl, ($C_2$ or $C_3$)-alkenyl, benzyl, phenethyl, 4-amino butyl or benzoyl methyl,

$R^2$ denotes hydrogen, ($C_1$ to $C_4$)-alkyl or benzyl,

X denotes phenyl, which can be monosubstituted or disubstituted or, in the case of methoxy, trisubstituted by ($C_1$ or $C_2$)-alkyl, ($C_1$ or $C_2$)-alkoxy, hydroxyl, fluorine, chlorine, bromine, amino, ($C_1$ to $C_4$)-alkyl-amino, di-($C_1$ to $C_4$)-alkylamino, nitro or methylenedioxy.

5. Compounds of the formula I as claimed in any of claims 1 to 4, characterized in that R denotes hydrogen, $R^1$ denotes methyl and X denotes phenyl.

6. Compounds of the formula I as claimed in any of claims 1 to 5, characterized in that R denotes hydrogen, $R^1$ denotes methyl, X denotes phenyl and $R^2$ denotes hydrogen or ethyl.

7. A compound of the formula I as claimed in claim 2, characterized in that the carboxyl group on the C atom in position 2 of the hexahydroindole system is oriented endo to the bicycle and the aforementioned C atom and the C atoms in the side chain marked with an asterisk each have the S configuration, and

n denotes 1,

R denotes hydrogen

$R^1$ denotes methyl

$R^2$ denotes ethyl

Y and Z each denote hydrogen and

X denotes phenyl.

8. A process for the preparation of compounds of the formula I as claimed in any of claims 1 to 7, which comprises a) reacting a compound of the formula II,

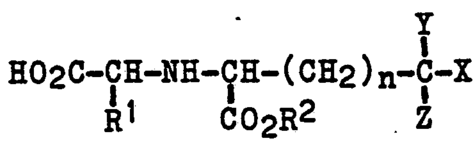

(II)

wherein n, $R^1$, $R^2$, X, Y and Z have the meaning as in formula I, with a compound of the formula III,

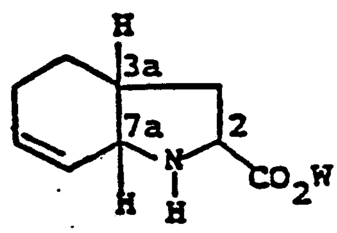

(III)

in which

W denotes hydrogen or a radical which can be split off by acid, and then optionally splitting off W and/or $R^2$ with formation of the free carboxyl group, or

b) for the preparation of compounds of the formula I in which Y and Z together denote oxygen,

$b_1$) reacting a compound of the formula IV,

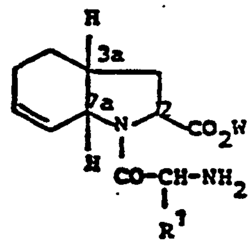

(IV)

in which $R^1$ has the meaning as in formula I and W has the meaning as in formula III, with a compound of the formula V,

$R^2O_2C\text{-}CH=CH\text{-}CO\text{-}X$ V

wherein $R^2$ and X have the meanings as in formula I, and then optionally splitting off W and/or $R^2$ with formation of the free carboxyl group, or

$b_2$) reacting a compound of the formula IV mentioned under $b_1$) with a compound of the formula VI, wherein $R^2$ has the meaning as in formula I, and with a compound of the formula VII,

$OHC\text{-}CO_2R^2$ $X\text{-}CO\text{-}CH_3$

(VI) (VII)

wherein X has the meaning as in formula I, and then optionally splitting off W and/or $R^2$ with formation of the free carboxyl group,

or

c) for the preparation of compounds of the formula I, in which Y and Z denote hydrogen, reacting a compound of the formula IV mentioned under bl) with a compound of the formula VIII,

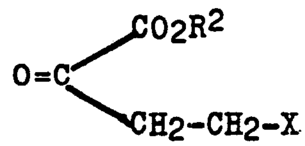

(VIII)

wherein $R^2$ and X have the meanings as in formula I, reducing the Schiff's bases obtained and then optionally splitting off W and/or $R^2$ with formation of the free carboxyl group or

d) for preparation of compounds of the formula I, in which Y denotes hydroxyl and Z denotes hydrogen, reducing, with a complex boranate or boraneamine complex, a compound of the formula I, in which Y and Z together denote oxygen,

and optionally converting the compounds of the formula I, in which R represents hydrogen, obtained according to a) to d), into esters of the formula I, wherein R denotes ($C_1$ to $C_6$)-alkyl or ($C_7$ to $C_9$)-aralkyl, and optionally converting the compound of the formula I into their physiologically acceptable salts.

9. A compound as claimed in any of claims 1 to 7 for the use as a medicament.

10. A pharmaceutical composition containing a compound as claimed in any of claims 1 to 7.

11. Compounds of the formula III,

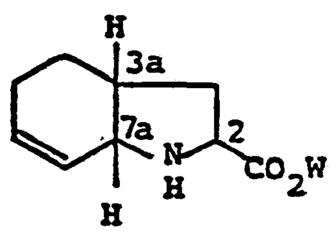

(III)

in which

the H atoms on the C atoms 3a and 7a have the cis configuration relative to one another and the group $-CO_2W$ on the C atom 2 is oriented exo or endo to the bicyclic ring system and wherein W denotes hydrogen or a radical which can be split off by acid.

12. A process for the preparation of compounds of the formula III as claimed in claim 11, which comprises reacting compounds of the formula IX,

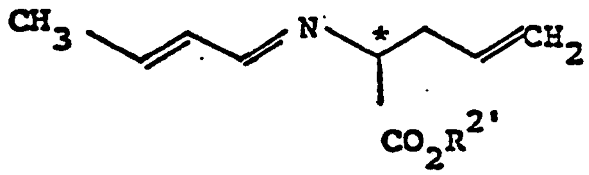

(IX)

in which

$R^{2'}$ denotes ($C_1$ to $C_8$)-alkyl or ($C_7$ to $C_8$)-aralkyl, with an acylating agent, which transfers the group $-CO-R^3$, wherein the radical $R^3$ represents ($C_1$ to $C_8$)-alkyl, ($C_5$ to $C_9$)-cycloalkyl, ($C_2$ to $C_8$)-alkenyl, $C_1$ to $C_8$)-alkoxy, aryl, aryloxy, aryl-($C_1$ to $C_4$)-alkyl or aryl-($C_1$ to $C_4$)-alkoxy, to give compounds of the formula X,

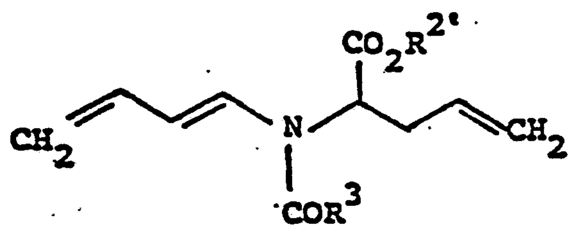

(X)

in which

$R^{2'}$ and $R^3$ have the foregoing meaning, cyclizing this to give a mixture of stereoisomeric compounds of the formula XI,

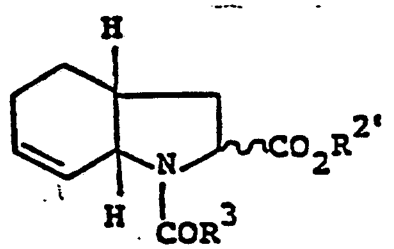

(XI)

in which

$R^{2'}$ and $R^3$ have the foregoing meaning and the hydrogen atoms on the bridgehead C atoms have the cis configuration,

hydrolyzing this mixture of stereoisomers, in which pairs of diastereomers are present, depending on whether the starting compounds of the formula IX had the R or S configuration or where racemic, of the formulae XIb and d or XIa and c, or mixtures of the formulae XIa-d, respectively

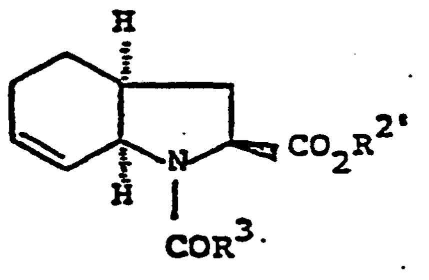

(XI a)

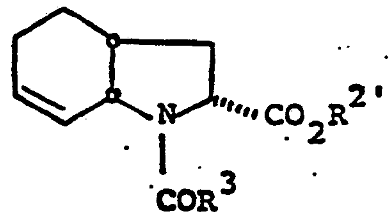

(XI b)

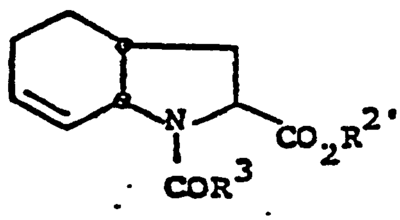

(XI c)

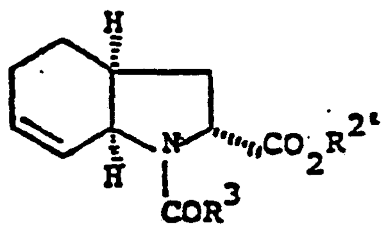

(XI d)

$R^{2'}$ and $R^3$ each have the foregoing meaning, hydrolyzing, if appropriate, after previous separation into enantiomers, pairs of diasteromers or racemates, to give compounds of the formula III as claimed in claim 11, in which W represents hydrogen and

if appropriate, esterifying the latter to give compounds of the formula III in which W represents a radical which can be split off by acid.

13. The use of a compound as claimed in claim 10 for the preparation of a compound as claimed in any of claims 1 to 7.

14. A compound as claimed in claim 9 for the use in combination with a diuretic.

15. A pharmaceutical composition containing a compound as claimed in any of claims 1 to 7 in combination with a diuretic.

16. A compounds as claimed in claim 9 or 14 for the use as a medicament for treating high blood pressure.

**Claims**

for the Contracting State: AT

1. A process for the preparation of compounds of the formula I,

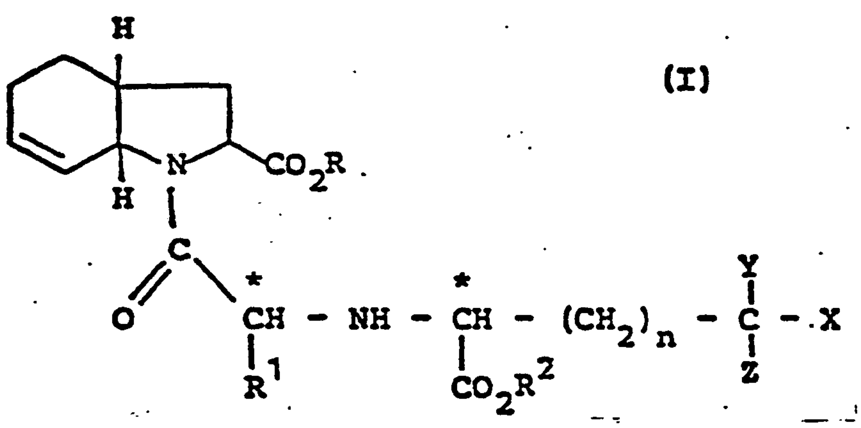

(I)

in which

n denotes 0 or 1,

R denotes hydrogen, ($C_1$ to $C_6$)-alkyl or aralkyl having 7 to 9 C atoms,

$R^1$ denotes hydrogen or ($C_1$ to $C_6$)-alkyl, which can optionally be substituted by amino, ($C_1$ to $C_4$)-acyl-amino or benzoylamino, ($C_2$ to $C_6$)-alkenyl, ($C_5$ to $C_9$)-cycloalkyl, ($C_5$ to $C_9$)-cycloalkenyl, ($C_5$ to $C_7$)-cycloalkyl-($C_1$ to

$C_4$)-alkyl, ($C_6$ to $C_{10}$)-aryl or partially hydrogenated ($C_6$ to $C_{10}$)-aryl, which each can be substituted by ($C_1$ to $C_4$)-alkyl, ($C_1$ or $C_2$)-alkoxy or halogen, ($C_6$ to $C_{10}$)-aryl-($C_1$ to $C_4$)-alkyl or ($C_7$ to $C_{11}$)-aroyl-$C_1$-alkyl, both of which can be substituted in the aryl radical as defined in the aforegoing, a monocyclic or bicyclic heterocyclic radical having 5 to 7 or 8 to 10 ring atoms respectively, of which 1 to 2 ring atoms represent sulfur or oxygen atoms and/or of which 1 to 4 ring atoms represent nitrogen atoms, or a side chain of a naturally occurring aminoacid,

$R^2$ denotes hydrogen, ($C_1$ to $C_6$)-alkyl, ($C_2$ to $C_6$)-alkenyl or aryl-($C_1$ to $C_4$)-alkyl,

Y denotes hydrogen or hydroxyl,

Z denotes hydrogen or

Y and Z together denote oxygen and

X denotes ($C_1$ to $C_6$)-alkyl, ($C_2$ to $C_6$)-alkenyl, ($C_5$ to $C_9$)-cycloalkyl, ($C_6$ to $C_{10}$)-aryl, which can be monosubstituted, disubstituted or trisubstituted by ($C_1$ to $C_4$)-alkyl, ($C_1$ to $C_4$)-alkoxy, hydroxyl, halogen, nitro, amino, ($C_1$ to $C_4$)-alkylamino, di-($C_1$ to $C_4$)-alkylamino or methylenedioxy, or 3-indolyl, and

its physiologically acceptable salts, which comprises

a) reacting a compound of the formula II,

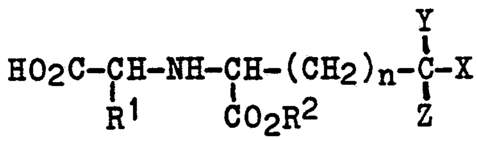

(II)

wherein n, $R^1$, $R^2$, X, Y and Z have the meaning as in formula I, with a compound of the formula III,

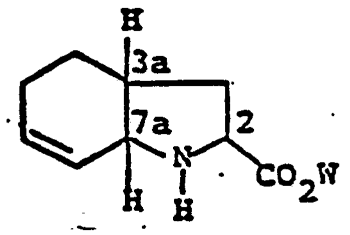

(III)

in which

W denotes hydrogen or a radical which can be split off by acid, and then optionally splitting off W and/or $R^2$ with formation of the free carboxyl group, or

b) for the preparation of compounds of the formula I in which Y and Z together denote oxygen,

$b_1$) reacting a compound of the formula IV,

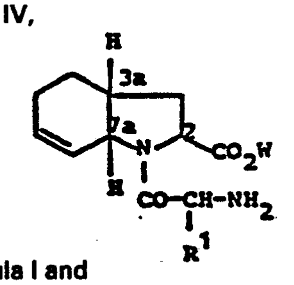

(IV)

in which $R^1$ has the meaning as in formula I and

W has the meaning as in formula III, with a compound of the formula V,

$R^2O_2C\text{-}CH{=}CH\text{-}CO\text{-}X$ V

wherein $R^2$ and X have the meanings as in formula I, and then optionally splitting off W and/or $R^2$ with formation of the free carboxyl group, or

$b_2$) reacting a compound of the formula IV mentioned under $b_1$) with a compound of the formula VI, wherein $R^2$ has the meaning as in formula I, and with a compound of the formula VII,

$OHC\text{-}CO_2R^2$ $X\text{-}CO\text{-}CH_3$

(VI) (VII)

wherein X has the meaning as in formula I, and then optionally splitting off W and/or $R^2$ with formation of the free carboxyl group,

or

c) for the preparation of compounds of the formula I, in which Y and Z denote hydrogen, reacting a compound of the formula IV mentioned under $b_1$) with a compound of the formula VIII,

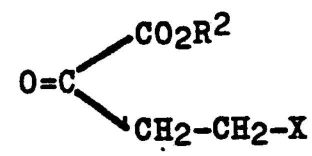

(VIII)

wherein $R^2$ and X have the meanings as in formula I, reducing the Schiff's bases obtained and then optionally splitting off W and/or $R^2$ with formation of the free carboxyl group or

d) for preparation of compounds of the formula I, in which Y denotes hydroxyl and Z denotes hydrogen, reducing, with a complex boranate or boraneamine complex, a compound of the formula I, in which Y and Z together denote oxygen,

and optionally converting the compounds of the formula I, in which R represents hydrogen, obtained according to a) to d), into esters of the formula I, wherein R denotes ($C_1$ to $C_6$)-alkyl or ($C_7$ to $C_9$)-aralkyl, and optionally converting the compound of the formula I into their physiologically acceptable salts.

2. A process as claimed in claim 1, in which compounds of the formula I are prepared, characterized in that the hydrogen atoms on the bridgehead C-atoms 3a and 7a have the cis configuration relative to one another.

3. A process as claimed in either of claims 1 or 2, in which compounds of the formula I are prepared, characterized in that the C atom in position 2 of the hexahydroindole system and the C atom in the side chain marked with an asterisk each have the S configuration.

4. A process as claimed in any of claims 1 to 3, in which compounds of the formula I are prepared, wherein n denotes 1,

R denotes hydrogen or ($C_1$ to $C_4$)-alkyl,

$R^1$ denotes hydrogen, ($C_1$ to $C_3$)-alkyl, ($C_2$ or $C_3$)-alkenyl, benzyl, phenethyl, 4-amino butyl or benzoyl methyl,

$R^2$ denotes hydrogen, ($C_1$ to $C_4$)-alkyl or benzyl,

X denotes phenyl, which can be monosubstituted or disubstituted or, in the case of methoxy, trisubstituted by ($C_1$ or $C_2$)-alkyl, ($C_1$ or $C_2$)-alkoxy, hydroxyl, fluorine, chlorine, bromine, amino, ($C_1$ to $C_4$)-alkyl-amino, di-($C_1$ to $C_4$)-alkylamino, nitro or methylenedioxy.

5. A process as claimed in any of claims 1 to 4, in which compounds of the formula I are prepared, characterized in that R denotes hydrogen, $R^1$ denotes methyl and X denotes phenyl.

6. A process as claimed in any of claims 1 to 5, in which a compound of the formula I is prepared, characterized in that R denotes hydrogen, $R^1$ denotes methyl, X denotes phenyl and $R^2$ denotes hydrogen or ethyl.

7. A process as claimed in claim 2, in which a compound of the formula I is prepared, characterized in that the carboxyl group on the C atom in position 2 of the hexahydroindole system is oriented endo to the bicycle and the aforementioned C atom and the C atoms in the side chain marked with an asterisk each have the S configuration, and

n denotes 1,

R denotes hydrogen

$R^1$ denotes methyl

$R^2$ denotes ethyl

Y and Z each denote hydrogen and

X denotes phenyl.

8. A process for preparing a compound of the formula I as claimed in any of claims 1 to 7 for the use as a medicament.

9. A process for making an agent containing a compound of the formula I, characterized in that the compound of the formula I is converted into a suitable form for administration.

10. A process for preparing compounds of the formula III,

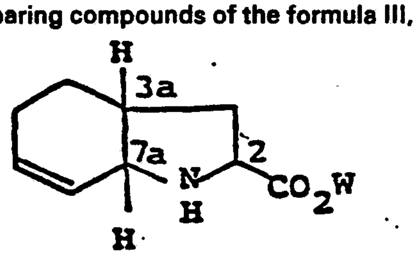

(III)

in which

the H atoms on the C atoms 3a and 7a have the cis configuration relative to one another and the group $-CO_2W$ on the C atom 2 is oriented exo or endo to the bicyclic ring system and wherein W denotes hydrogen or a radical which can be split off by acid, which comprises reacting compounds of the formula IX,

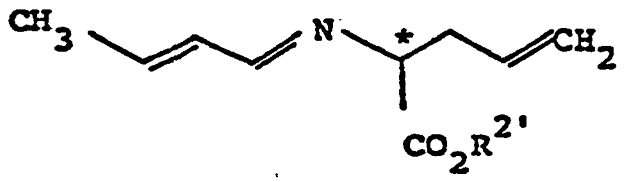

(IX)

in which

$R^{2'}$ denotes ($C_1$ to $C_6$) -alkyl or ($C_7$ to $C_8$)-aralkyl, with an acylating agent, which transfers the group -CO-$R^3$, wherein the radical $R^3$ represents ($C_1$ to $C_6$)-alkyl, ($C_5$ to $C_9$)-cycloalkyl, ($C_2$ to $C_6$)-alkenyl, $C_1$ to $C_6$)-alkoxy, aryl, aryloxy, aryl-($C_1$ to $C_4$)-alkyl or aryl-($C_1$ to $C_4$)-alkoxy, to give compounds of the formula X,

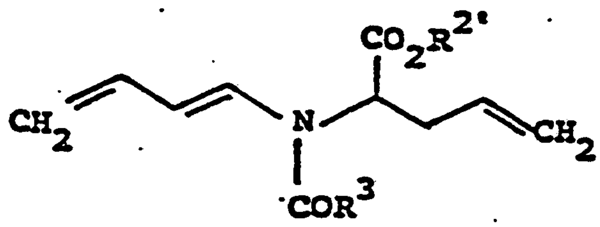

(X)

in which

$R^{2'}$ and $R^3$ have the foregoing meaning, cyclizing this to give a mixture of stereoisomeric compounds of the formula XI,

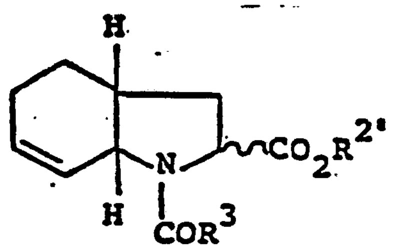

(XI)

in which

$R^{2'}$ and $R^3$ have the foregoing meaning and the hydrogen atoms on the bridgehead C atoms have the cis configuration,

hydrolyzing this mixture of stereoisomers, in which pairs of diastereomers are present, depending on whether the starting compounds of the formula IX had the R or S configuration or were racemic, of the formulae XIb and d or XIa and c, or mixtures of the formulae XIa-d, respectively

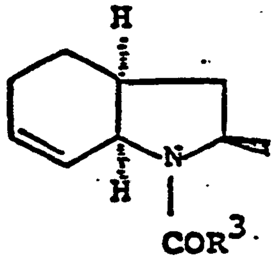

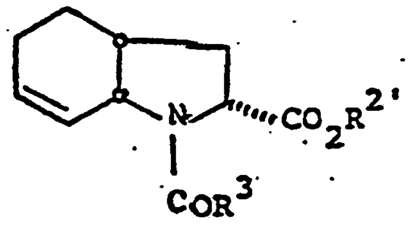

(XI a) (XI b)

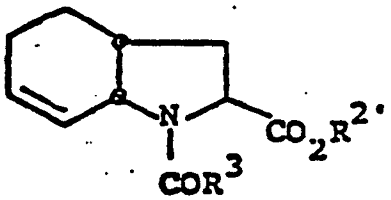

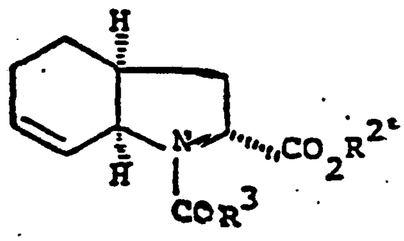

(XI c) (XI d)

in which

$R^{2'}$ and $R^3$ each have the foregoing meaning, hydrolyzing, if appropriate, after previous separation into enantiomers, pairs of diasteromers or racemates, to give compounds of the formula III in which W represents hydrogen and

if appropriate, esterifying the latter to give compounds of the formula III in which W represents a radical which can be split off by acid.

11. A process for preparing a compound of the formula I as claimed in any of claim 1 to 7 for the use as a medicament in combination with a diuretc.

12. A process for preparing an agent, containing a compound of the formula I in combination with a diuretic, characterized in that the compound of the formula I together with the diuretic is converted into a suitable form for administration.

**Revendications**

pour les Etats contractants: BE, CH, DE, FR, GB, IT, LI, LU, NL, SE

1.Composés de formule:

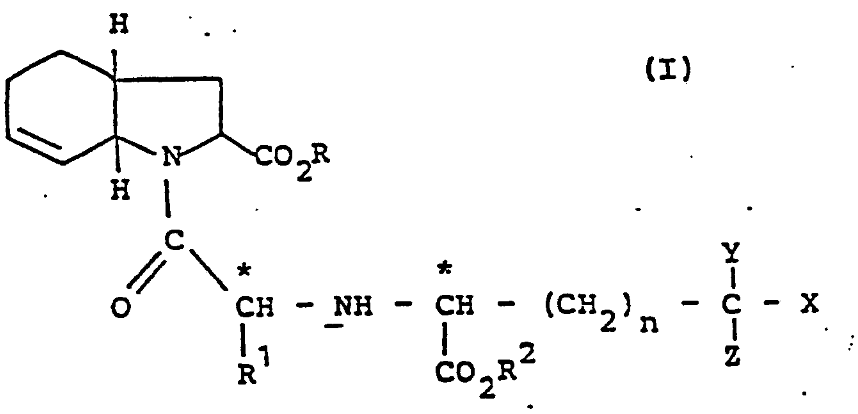

(I)

dans laquelle

n est 0 ou 1

R représente l'hydrogène ou un groupe alcoyle en $C_1$-$C_6$ ou aralcoyle ayant de 7 à 9 atomes de carbone;

$R^1$ représente l'hydrogène ou un groupe alcoyle en $C_1$-$C_6$, qui peut être substitué par un groupe amino, acyl(en $C_1$-$C_4$)-amino ou benzoylamino, un groupe alcényle en $C_2$-$C_6$, cycloalcoyle en $C_5$-$C_9$, cycloalcényle en

$C_5$-$C_9$, cycloalcoyl(en $C_5$-$C_7$)-alcoyle (en $C_1$-$C_4$), aryle en $C_6$-$C_{10}$ ou aryle en $C_6$-$C_{10}$ partiellement hydrogéné, qui peut être substitué dans chaque cas par un groupe alcoyle en $C_1$-$C_4$, alcoxy en $C_1$ ou $C_2$ ou par un halogène, un groupe aryl(en $C_6$-$C_{10}$)-alcoyle (en $C_1$-$C_4$) ou aroyl(en $C_7$-$C_{11}$)-alcoyle (en $C_1$) qui peuvent tous les deux être substitués sur le radical aryle comme défini précédemment, un radical hétérocyclène mono- ou bicyclique avec de 5 à 7 ou de 8 à 10 atomes dans le noyau, de 1 à 2 atomes du noyau étant des atomes de soufre ou d'oxygène et/ou de 1 à 4 atomes dans le noyau étant constitués par des atomes d'azote, ou une chaîne latérale d'un acide aminé d'origine naturelle

$R^2$ représente l'hydrogène ou un groupe alcoyle en $C_1$-$C_6$, alcényle en $C_2$-$C_6$ ou aryl-alcoyle (en $C_1$-$C_4$);
Y représente l'hydrogène ou un groupe hydroxy,
Z représente l'hydrogène ou
Y et Z représentent ensemble l'oxygène et
X représente un groupe alcoyle en $C_1$-$C_6$, alcényle en $C_2$-$C_6$, cycloalcoyle en $C_5$-$C_9$, aryle en $C_6$-$C_{10}$ qui peut être mono-, di- ou trisubstitué par un groupe alcoyle en $C_1$-$C_4$, alcoxy en $C_1$-$C_4$, hydroxy, un halogène, un groupe nitro, amino, alcoyl(en $C_1$-$C_4$)-amino, di-alcoyl(en $C_1$-$C_4$)-amino ou méthylènedioxy, ou un groupe 3-indolyle;
et leurs sels physiologiquement acceptables.

2. Composés de formule I selon la revendication 1, caractérisés en ce que les atomes d'hydrogène sur les atomes de carbone têtes de pont 3a et 7a sont en configuration cis l'un par rapport à l'autre.

3. Composés de formule I selon l'une des revendications 1 et 2, caractérisés en ce que l'atome de carbone en position 2 du système hexahydroindole, ainsi que les atomes de carbone de la chaîne latérale, marqués d'un astérisque, montrent chacun la configuration S.

4. Composés de formule I selon l'une quelconque des revendications 1 à 3, caractérisés en ce que:
n est 1,
R représente l'hydrogène ou un groupe alcoyle en $C_1$-$C_4$,
$R^1$ représente l'hydrogène, un groupe alcoyle en $C_1$-$C_3$, alcényle en $C_2$ ou $C_3$, benzyle, phénéthyle, 4-amino-butyle ou benzoyl-méthyle,
$R^2$ représente l'hydrogène ou un groupe alcoyle en $C_1$-$C_4$ ou benzyle,
X représente un groupe phényle qui peut être mono- ou disubstitué par un groupe alcoyle en $C_1$ ou $C_2$, alcoxy en $C_1$ ou $C_2$, hydroxy, fluoro, chloro, bromo, amino, alcoyl: (en $C_1$-$C_4$)-amino, dialcoyl(en $C_1$-$C_4$)-amino, nitro ou méthylènedioxy, ou trisubstitué dans le cas d'un groupe méthoxy.

5. Composés de formule I selon l'une quelconque des revendications 1 à 4, caractérisés en ce que R représente l'hydrogène, $R^1$ représente un groupe méthyle et X représente un groupe phényle.

6. Composés de formule I selon l'une quelconque des revendications 1 à 5, caractérisés en ce que R représente l'hydrogène, $R^1$ représente un groupe méthyle, X représente un groupe phényle et $R^2$ représente l'hydrogène ou un groupe éthyle.

7. Composé de formule I selon la revendication 2, caractérisé en ce que le groupe carboxy sur l'atome de carbone en position 2 du système hexahydroindole est orienté en position endo par rapport au système bicyclique et l'atome de carbone précédemment nommé ainsi que les atomes de carbone de la chaîne latérale, marqués d'un astérisque, montrent chacun une configuration S,
n est 1,
R représente l'hydrogène,
$R^1$ représente un groupe méthyle,
$R^2$ représente un groupe éthyle,
Y et Z représentent chacun l'hydrogène et
X représente un groupe phényle.

8. Procédé de préparation des composés de formule I selon l'une quelconque des revendications 1 à 7, caracterise en ce que
a) on fait réagir un composé de formule II:

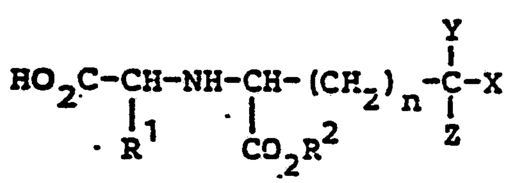

(II)

dans laquelle n, $R^1$, $R^2$, X, Y et Z ont les significations indiquées pour la formule I, avec un composé de formule III:

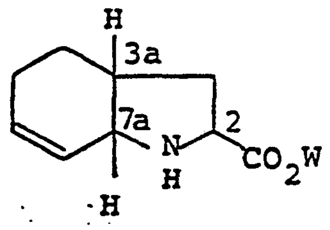

(III)

dans laquelle
W représente l'hydrogène ou un radical éliminable par un acide,
puis on élimine éventuellement W et/ou $R^2$ pour former les groupes carboxy libres, ou
b) pour la préparation des composés de formule I dans lesquels Y et Z représentent ensemble l'oxygène, $b_1$) on fait réagir un composé de formule IV:

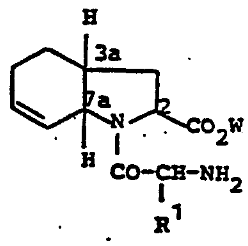

(IV)

dans laquelle $R^1$ a la signification indiquée pour la formule I et W a la signification indiquée pour la formule III, avec un composé de formule V
$R^2O_2C-CH=CH-CO-X$ (V)
dans laquelle $R^2$ et X ont les significations indiquées pour la formule I, puis on élimine éventuellement W et/ou $R^2$ en formant les groupes carboxy libres, ou
$b_2$) on fait réagir un composé de formule IV mentionnée en $b_1$) avec un composé de formule générale VI dans laquelle $R^2$ a la signification indiquée pour la formule I et avec un composé de formule générale VII:
$OHC-CO_2R^2$ $X-CO-CH_3$
(VI) (VII)
dans laquelle X a la signification indiquée pour la formule I, puis éventuellement on élimine W et/ou $R^2$ pour former les groupes carboxy libres, ou
c) pour la préparation de composés de formule I dans laquelle Y et Z représentent chacun l'hydrogène, on fait réagir un composé de formule IV mentionnée en $b_1$) avec un composé de formule VIII:

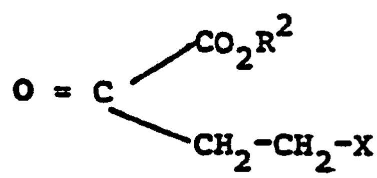

(VIII)

dans laquelle $R^2$ et X ont les significations indiquées pour la formule I, on réduit les bases de Schiff obtenues, puis éventuellement on élimine W et/ou $R^2$ pour former les groupes carboxy libres, ou
d) pour la préparation de composés de formule I dans lesquels Y représente un groupe hydroxy et Z représente l'hydrogène, on réduit un composé de formule I dans lequel Y et Z représentent ensemble l'oxygène, avec un boranate complexe ou un complexe borane-amine,
et on convertit éventuellement les composés de formule I obtenus selon a) à d), dans lesquels R représente l'hydrogène, en esters de formule I dans lesquels R représente un groupe alcoyle en $C_1$-$C_6$ ou aralcoyle en $C_7$-$C_9$ et on convertit éventuellement les composés de formule I en leurs sels physiologiquement acceptables.
9. Composé selon l'une quelconque des revendications 1 à 7, à utiliser en tant que médicament.
10. Médicament contenant un composé selon l'une quelconque des revendications 1 à 7.
11. Composés de formule III:

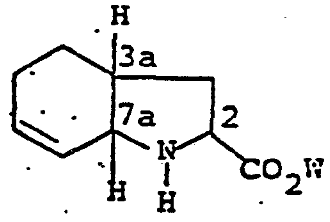

(III)

dans laquelle les atomes d'hydrogène sur les atomes de carbone 3a et 7a sont en configuration cis l'un par rapport à l'autre et le groupe $-CO_2W$ sur l'atome de carbone 2 est orienté en position exo ou endo par rapport

au système bicyclique et dans laquelle W représente un atome d'hydrogène ou un radical éliminable par un acide.

12. Procédé de préparation de composés de formule III selon la revendication 11, caractérisé en ce que: on fait réagir des composés de formule IX:

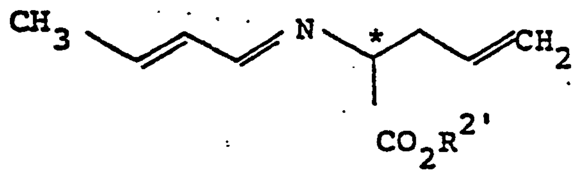

(IX)

dans laquelle $R^2$ représente un groupe alcoyle en $C_1$-$C_8$ ou aralcoyle en $C_7$ ou $C_8$, avec des agents d'acylation qui portent le groupe $-COR^3$ dans lequel le radical $R^3$ représente un groupe alcoyle en $C_1$-$C_6$, cycloalcoyle en $C_5$-$C_9$, alcényle en $C_2$-$C_6$, alcoxy en $C_1$-$C_6$, aryle, aryloxy, arylalcoyle(en $C_1$-$C_4$) ou aryl-alcoxy (en $C_1$-$C_4$), pour obtenir des composés de formule X:

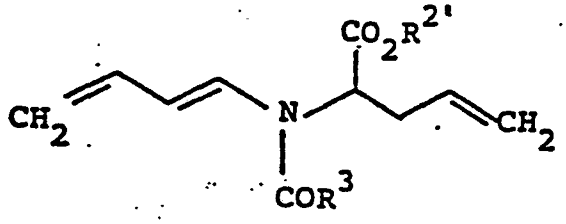

(X)

dans laquelle $R^{2'}$ et $R^3$ ont les significations précédentes, on cyclise ces composés en un mélange de composés stéréoisomères de formule XI:

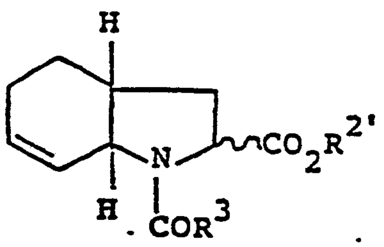

(XI)

dans laquelle $R^{2'}$ et $R^3$ ont les significations précédentes, et les atomes d'hydrogène sur les atomes de carbone têtes de pont sont en configuration cis, on saponifie ce mélange de stéréoisomères dans lequel, selon qu'on est parti de composés de formule IX en configuration R, S ou de composés de formule IX racémiques, se trouvent des paires de diastéréomères de formules XIb et d ou XIa et c, ou des mélanges de formules IXa à d

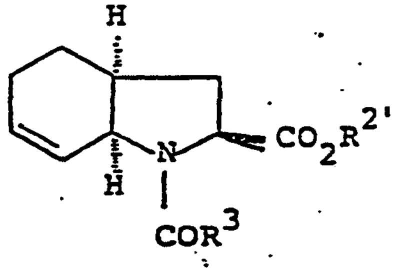

(XI a)

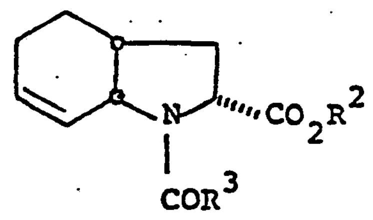

(XI b)

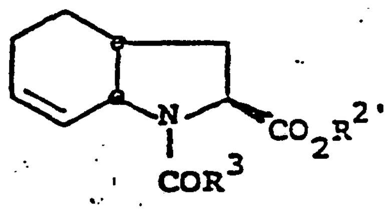

(XI c)

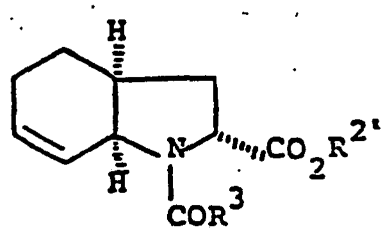

(XI d)

dans lesquelles $R^{2'}$ et $R^3$ ont chacun les significations précédentes, éventuellement après séparation préalable en énantiomères, paires de diastéréomères ou racémates, en des composés de formule III selon la revendication 11, dans lesquels W représente l'hydrogène, et

on estérifie éventuellement ces composés en composés de formule III, dans lesquels W représente un radical éliminable par un acide.

13. Utilisation d'un composé selon la revendication 11 pour la préparation d'un composé selon l'une quelconque des revendications 1 à 7.

14. Composé selon la revendication 9, à utiliser en association avec un diurétique en tant que médicament.

15. Médicament contenant un composé selon l'une quelconque des revendications 1 à 7, en association avec un diurétique.

16. Composé selon l'une des revendiations 9 et 14 à utiliser en tant que médicament dans le traitement de l'hypertension.

**Revendications**

pour l'Etat contractant: AT

1. Procédé de préparation de composés de formule:

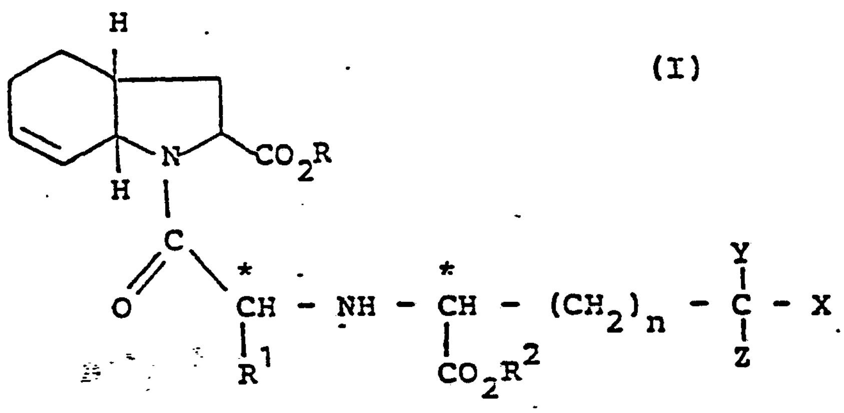

(I)

dans laquelle

n est 0 ou 1:

R représente l'hydrogène ou un groupe alcoyle en $C_1$-$C_6$ ou aralcoyle ayant de 7 à 9 atomes de carbone;

$R^1$ représente l'hydrogène ou un groupe alcoyle en $C_1$-$C_6$, qui peut être éventuellement substitué par un

groupe amino, acyl (en $C_1$-$C_4$)-amino, ou benzoylamino, un groupe alcényle en $C_2$-$C_6$, cycloalcoyle en $C_5$-$C_9$, cycloalcényle en $C_5$-$C_9$, cycloalcoyl(en $C_5$-$C_7$)-alcoyle (en $C_1$-$C_4$) aryle en $C_6$-$C_{10}$ ou aryle en $C_6$-$C_{10}$ partiellement hydrogéné, qui peut être substitué dans chaque cas par un groupe alcoyle en $C_1$-$C_4$, alcoxy en $C_1$ ou $C_2$ ou par un halogène, un groupe aryl(en $C_6$-$C_{10}$)-alcoyle (en $C_1$-$C_4$) ou aroyl(en $C_7$-$C_{11}$)-alcoyle (en $C_1$) qui peuvent tous les deux être substitués sur le radical aryle comme défini précédemment, un radical hétérocyclène mono- ou bicyclique avec de 5 à 7 ou de 8 à 10 atomes dans le noyau, de 1 à 2 atomes du noyau étant des atomes de soufre ou d'oxygène et/ou de 1 à 4 atomes dans le noyau étant constitués par des atomes d'azote, ou une chaîne latérale d'un acide aminé d'origine naturelle;

$R^2$ représente l'hydrogène ou un groupe alcoyle en $C_1$-$C_6$, alcényle en $C_2$-$C_6$ ou aryl-alcoyle (en $C_1$-$C_4$);

Y représente l'hydrogène ou un groupe hydroxy,

Z représente l'hydrogène ou

Y et Z représentent ensemble l'oxygène et

X représente un groupe alcoyle en $C_1$-$C_6$, alcényle en $C_2$-$C_6$, cycloalcoyle en $C_5$-$C_9$, aryle en $C_6$-$C_{10}$ qui peut être mono-, di- ou trisubstitué par un groupe alcoyle en $C_1$-$C_4$, alcoxy en $C_1$-$C_4$, hydroxy, un halogène, un groupe nitro, amino, alcoyl(en $C_1$-$C_4$)-amino, di-alcoyl(en $C_1$-$C_4$)-amino ou méthylènedioxy, ou un groupe 3-indolyle;

et de leurs sels physiologiquement acceptables, caractérisé en ce que:

a) on fait réagir un composé de formule II:

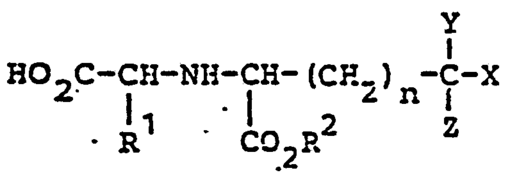

(II)

dans laquelle n, $R^1$, $R^2$, X, Y et Z ont les significations indiquées pour la formule I, avec un composé de formule III:

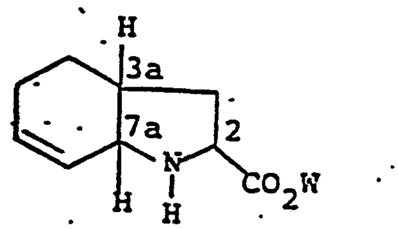

(III)

dans laquelle:

W représente l'hydrogène ou un radical éliminable par un acide,

puis on élimine éventuellement W et/ou R pour former les groupes carboxy libres, ou

b) pour la préparation des composés de formule I dans lesquels Y et Z représentent ensemble l'oxygène,

$b_1$) on fait réagir un composé de formule IV:

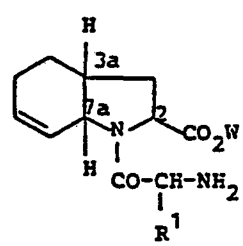

(IV)

dans laquelle $R^1$ a la signification indiquee pour la formule I et W a la signification indiquée pour la formule III, avec un composé de formule V:

$R^2O_2C$-CH=CH-CO-X (V)

dans laquelle $R^2$ et X ont les significations indiquées pour la formule I, puis on élimine éventuellement W et/ou $R^2$ en formant les groupes carboxy libres, ou

$b_2$) on fait réagir un composé de formule IV mentionnée en $b_1$) avec un composé de formule générale VI dans laquelle $R^2$ a la signification indiquée pour la formule I et avec un composé de formule générale VII:

OHC-$CO_2R^2$ X-CO-$CH_3$

(VI) (VII)

dans laquelle X a la signification indiquée pour la formule I, puis éventuellement on élimine W et/ou $R^2$ pour former les groupes carboxy libres, ou

c) pour la préparation de composés de formule I dans laquelle Y et Z représentent chacun l'hydrogène, on fait réagir un composé de formule IV mentionnée en $b_1$) avec un composé de formule VIII:

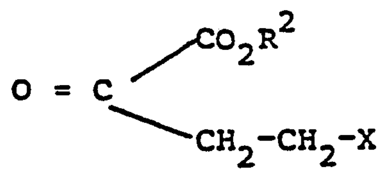

(VIII)

dans laquelle $R^2$ et X ont les significations indiquées pour la formule I, on réduit les bases de Schiff obtenues, puis éventuellement on élimine W et/ou $R^2$ pour former les groupes carboxy libres, ou

d) pour la préparation de composés de formule I dans lesquels Y représente un groupe hydroxy et Z représente l'hydrogène, on réduit un composé de formule I dans lequel Y et Z représentent ensemble l'oxygène, avec un boranate complexe ou un complexe borane-amine, et on convertit éventuellement les composés de formule I obtenus selon a) à d), dans lesquels R représente l'hydrogène, en esters de formule I dans lesquels R représente un groupe alcoyle en $C_1$-$C_6$ ou aralcoyle en $C_7$-$C_9$, et on convertit éventuellement les composés de formule I en leurs sels physiologiquement acceptables.

2. Procédé selon la revendication 1, caractérisé en ce que les atomes d'hydrogène sur les atomes de carbone têtes de pont 3a et 7a sont en configuration cis l'un par rapport à l'autre.

3. Procédé selon l'une des revendications 1 et 2, caractérisé en ce que l'atome de carbone en position 2 du système hexahydroindole, ainsi que les atomes de carbone de la chaîne latérale, marqués d'un astérisque, montrent chacun la configuration S.

4. Procédé selon l'une quelconque des revendications 1 à 3, caractérisé en ce que:

n est 1,

R représente l'hydrogéne ou un groupe alcoyle en $C_1$-$C_4$,

$R^1$ représente l'hydrogéne, un groupe alcoyle en $C_1$-$C_3$, alcényle en $C_2$ ou $C_3$, benzyle, phénéthyle, 4-amino-butyle ou benzoyl-méthyle,

$R^2$ représente l'hydrogéne ou un groupe alcoyle en $C_1$-$C_4$ ou benzyle,

X représenté un groupe phényle qui peut être mono- ou disubstitué par un groupe alcoyle en $C_1$ ou $C_2$, alcoxy en $C_1$ ou $C_2$, hydroxy, fluoro, chloro, bromo, amino, alcoyl(en $C_1$-$C_4$)-amino, dialcoyl(en $C_1$-$C_4$)-amino, nitro ou méthylènedioxy, ou trisubstitué dans le cas d'un groupe méthoxy.

5. Procédé selon l'une quelconque des revendications 1 à 4, caractérisé en ce que R représente l'hydrogène, $R^1$ représente un groupe méthyle et X représente un groupe phényle.

6. Procédé selon l'une quelconque des revendications 1 à 5, caractérisé en ce que R représente l'hydrogène, $R^1$ représente un groupe méthyle, X représente un groupe phényle et $R^2$ représente l'hydrogène ou un groupe éthyle.

7. Procédé selon la revendication 2, caractérisé en ce que le groupe carboxy sur l'atome de carbone en position 2 du système hexahydroindole est orienté en position endo par rapport au système bicyclique et l'atome de carbone précédemment nommé ainsi que les atomes de carbone de la chaîne latérale, marqués d'un astérisque, montrent chacun une configuration 8,

n est 1,

R représente l'hydrogène,

$R^1$ représente un groupe méthyle,

$R^2$ représente un groupe èthyle,

Y et Z représentent chacun l'hydrogène et

X représente un groupe phényle.

8. Procédé de préparation d'un composé de formule I selon l'une quelconque des revendications 1 à 7 à utiliser en tant que médicament.

9. Procédé de préparation d'une composition contenant un composé de formule I, caracterisé en ce qu'on met le composé de formule I sous une forme appropriée.

10. Procédé de préparation de composés de formule III:

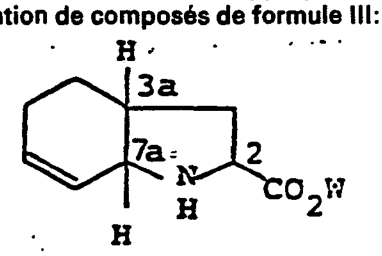

(III)

dans laquelle les atomes d'hydrogène sur les atomes de carbone 3a et 7a sont en configuration cis l'un par rapport à l'autre et le groupe $-CO_2W$ sur l'atome de carbone 2 est orienté en position exo ou endo par rapport au système bicyclique et dans laquelle W représente un atome d'hydrogène ou un radical éliminable par un acide caractérisé en ce que: on fait réagir des composés de formule IX:

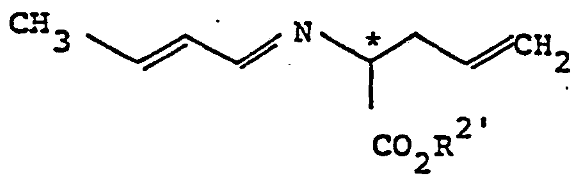

(IX)

dans laquelle $R^{2'}$ représente un groupe alcoyle en $C_1$-$C_6$ ou aralcoyle en $C_7$ ou $C_8$, avec des agents d'acylation qui portent le groupe -$COR^3$ dans lequel le radical $R^3$ représente un groupe alcoyle en $C_1$-$C_6$, cycloalcoyle en $C_5$-$C_9$, alcényle en $C_2$-$C_6$, alcoxy en $C_1$-$C_6$, aryle, aryloxy, arylalcoyle(en $C_1$-$C_4$) ou aryl-alcoxy (en $C_1$-$C_4$), pour obtenir des composés de formule X:

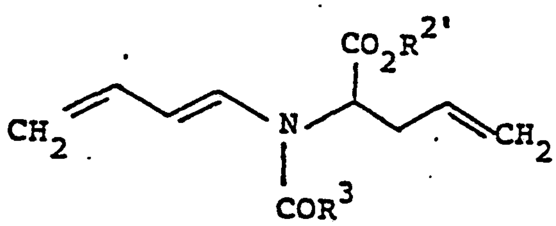

(X)

dans laquelle $R^{2'}$ et $R^3$ ont les significations précédentes, on cyclise ces composés en un mélange de composés stéréoisomères de formule XI:

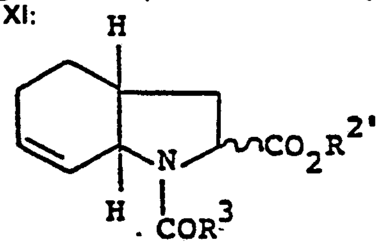

(XI)

dans laquelle $R^{2'}$ et $R^3$ ont les significations précédentes, et les atomes d'hydrogène sur les atomes de carbone têtes de pont sont en configuration cis, on saponifie ce mélange de stéréoisomères dans lequel, selon qu'on est parti de composés de formule IX en configuration R, S ou de composés de formule IX racémiques, se trouvent des paires de diastéréomères de formules XIb et d ou XIa et c, ou des mélanges de formules IXa à d:

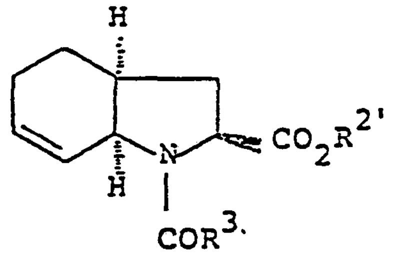

(XI a)

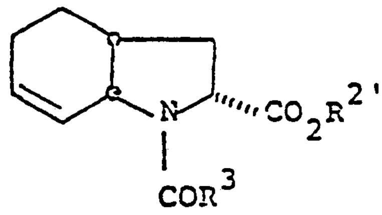

(XI b)

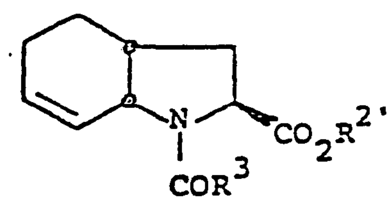

(XI c)

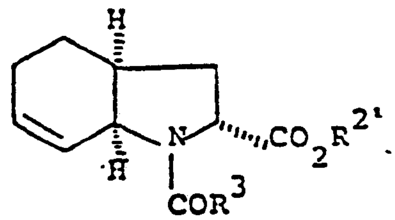

(XI d)

dans lesquelles $R^{2'}$ et $R^3$ ont chacun les significations précédentes, éventuellement aprés séparation préalable en enantioméres, paires de diastéréomères ou racémates, en des composés de formule III selon la revendication 11, dans lesquels W représente l'hydrogène, et

on estérifie éventuellement ces composés en composés de formule III, dans lesquels W représente un radical éliminable par un acide.

11. Procédé de préparation d'un composé de formule I selon l'une quelconque des revendications 1 à 7, à utiliser en association avec un diurétique, en tant que médicament.

12. Procédé de préparation d'une composition contenant un composé de formule I asscocié à in diurétique, caractérisé en ce qu'on met le composé de formule I, en association avec un diurétique, sous une forme appropriée.